(19) Europäisches Patentamt
European Patent Office
Office européen des brevets

(11) EP 3 607 044 B1

(12) **EUROPEAN PATENT SPECIFICATION**

(45) Date of publication and mention of the grant of the patent:
**13.11.2024 Bulletin 2024/46**

(21) Application number: **18715715.1**

(22) Date of filing: **06.04.2018**

(51) International Patent Classification (IPC):
***C11D 3/386*** *(2006.01)* ***C12N 9/22*** *(2006.01)*
***C11D 1/00*** *(2006.01)*

(52) Cooperative Patent Classification (CPC):
**C11D 3/38636; C12N 9/22;** C11D 2111/12

(86) International application number:
**PCT/EP2018/058858**

(87) International publication number:
**WO 2018/185285 (11.10.2018 Gazette 2018/41)**

(54) **CLEANING COMPOSITIONS AND USES THEREOF**

REINIGUNGSMITTELZUSAMMENSETZUNGEN UND VERWENDUNGEN DAVON

COMPOSITIONS DÉTERGENTES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.04.2017 EP 17165317**

(43) Date of publication of application:
**12.02.2020 Bulletin 2020/07**

(73) Proprietor: **Novozymes A/S**
**2880 Bagsvaerd (DK)**

(72) Inventors:
- **GORI, Klaus**
  **2880 Bagsvaerd (DK)**
- **BARRIENTOS, Fabian**
  **2880 Bagsvaerd (DK)**
- **GJERMANSEN, Morten**
  **2880 Bagsvaerd (DK)**

(74) Representative: **NZ EPO Representatives**
**Krogshoejvej 36**
**2880 Bagsvaerd (DK)**

(56) References cited:
**WO-A1-2013/003659** **WO-A1-2016/176241**
**WO-A1-2016/176280** **WO-A2-01/46512**
**WO-A2-2008/153815**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Reference to a Sequence Listing

This application contains a Sequence Listing in computer readable form.

### Background of the Invention

The present invention relates to compositions such as cleaning compositions comprising a mix of enzymes. The invention further relates to use of compositions comprising such enzymes in cleaning processes and/or for deep cleaning of organic soiling.

### Description of the Related Art

Enzymes have been used in detergents for decades. Usually a cocktail of various enzymes is added to detergent compositions. The enzyme cocktail often comprises various enzymes, wherein each enzyme targets it specific substrate e.g. amylases are active towards starch stains, proteases on protein stains and so forth. Textiles surface and hard surfaces, such as dishes or the inner space of a laundry machine enduring several wash cycles, become soiled with many different types of soiling which may compose of proteins, grease, starch etc. One type of soiling may be organic matter, such as biofilm, EPS, etc. Organic matter composes different molecules such as polysaccharides, extracellular DNA (eDNA), RNA and proteins. Some organic matter composes an extracellular polymeric matrix, which may be sticky or glueing, which when present on textile, attracts soils and may course redeposition or backstaining of soil resulting in a greying of the textile. Additionally, organic matters such as biofilms often cause malodor issue as various malodor molecules can be adhered by the polysaccharides, extracellular DNA (eDNA), RNA and proteins in the complex extracellular matrix and be slowly released out to cause consumer noticeable malodor issue. There is still a need for cleaning compositions, which effectively prevent, reduce or remove components of organic soiling, an effect described in the present application as "deep cleaning". The present invention provides new compositions fulfilling such need.

WO 2016/176241 discloses a cleaning composition comprising a nuclease enzyme and a surfactant system comprising an anionic surfactant and a nonionic surfactant. WO 2016/176280 discloses a method of treating a hydrophobically-modified textile using an aqueous solution comprising a nuclease enzyme. The nuclease sequences disclosed in these documents have a low sequence identity to the sequences disclosed herein.

### Summary of the Invention

The present invention relates to a cleaning composition comprising a DNase, an RNase and a cleaning component as defined in the claims. The cleaning compositions may comprise at least 0.001 ppm DNase and at least 0.001 ppm RNase and a cleaning component, wherein the cleaning component is selected from

a. 0.1 to 15 wt% of at least one a surfactant;
b. 0.5 to 20 wt% of at least one builder; and
c. 0.01 to 10 wt% of at least one bleach component

The invention further relates to a method of deep cleaning of an item, comprising the steps of:

a) contacting the item with a cleaning composition comprising a DNase, an RNase and a cleaning component; and
b) optionally rinsing the item, wherein the item is preferably a textile.

The method of deep cleaning of an item may comprise the steps of: a) contacting the item with a solution comprising an enzyme mixture comprising a DNase and an RNase and optionally a protease; and a cleaning component, wherein the cleaning component is selected from 0.1 to 15 wt% of at least one a surfactant; 0.5 to 20 wt% of at least one builder; and 0.01 to 10 wt% of at least one bleach component; and b) and optionally rinsing the item, wherein the item is preferably a textile.

### Detailed Description of the Invention

Various enzymes are applied in cleaning processes each targeting specific types of soiling such as protein, starch and grease soiling. Enzymes are now standard ingredients in detergents for laundry and dish wash. The effectiveness of these commercial enzymes provides detergents which removes much of the soiling. However, organic matters

such as EPS (extracellular polymeric substance) comprised in much biofilm constitute a challenging type of soiling due to the complex nature of such organic matters. None of the commercially available cleaning compositions effectively remove or reduce EPS and/or biofilm related soiling. The present invention provides compositions comprising a blend of at least one DNase and an RNase which effectively reduce of remove components of organic soiling. Biofilm may be produced when a group of microorganisms' cells stick to each other or stick to a surface, such as a textile, dishware or hard surface or another kind of surface. These adherent cells are frequently embedded within a self-produced matrix of extracellular polymeric substance (EPS), which constitute 50% to 90% of the biofilm's total organic matter. EPS is mostly composed of polysaccharides (exopolysaccharides) and proteins, but include other macro-molecules such as eDNA, RNA, lipids and other organic substances. Organic matter like biofilm may be sticky or glueing, which when present on textile, may give rise to redeposition or backstaining of soil resulting in a greying of the textile. Another drawback of organic matter e.g. biofilm is the malodor as various malodor related molecules are often associated with organic matter e.g. biofilm. Further, when dirty laundry items are washed together with less dirty laundry items the dirt present in the wash liquor tend to stick to organic matter e.g. biofilm or biofilm components thus, hereof the laundry item is more "soiled" after wash than before wash. This is effect may also be termed re-deposition.

Examples of useful DNases and RNase are mentioned below in the section "Nucleases".

The compositions of the invention comprising a blend of DNase and RNase are effective in reducing or removing organic components and soiling from organic matter.

**Nucleases**

Nucleases is a common term for enzymes capable of cleaving the phosphodiester bonds between monomers of nucleic acids. Exonucleases digest nucleic acids from the ends. Endonucleases act on regions in the middle of target molecules. Nucleases are further subcategorized as deoxyribonucleases acting on DNA and ribonucleases acting on RNA.

The present invention relates to cleaning compositions comprising a blend of at least one polypeptide having DNase activity (DNase) and at least one polypeptide having RNase activity (RNase).

Some nucleases are DNases having only DNase activity and some DNases have minor RNase activity but are still characterized as being DNase and likewise for the RNases. The compositions of the invention comprise nucleases with one and/or both activities. One embodiment relates to compositions comprising a DNase without RNase activity and an RNase without DNase activity. One embodiment relates to compositions comprising nucleases which exhibit both DNase and RNase activity.

One example of a nuclease having both DNase and RNase activity is the Endonuclease from *Serratia marcescens* commercially available under the name Benzonase® (available from Sigma-Aldrich). Thus, the composition of the invention may include nucleases selected from E.C. 3.1.30.1 or E.C. 3.1.30.2.

Some suitable nucleases to be included in a composition of the invention are listed below.

<u>Polypeptides having DNase activity (DNase)</u>

The term "DNase" means a polypeptide with DNase (deoxyribonuclease) activity that catalyzes the hydrolytic cleavage of phosphodiester linkages in a DNA backbone, thus degrading DNA. Exodeoxyribonucleases cut or cleave residues at the end of the DNA backbone whereas endo-deoxyribonucleases cleave or cut within the DNA backbone. A DNase may cleave only doublestranded DNA or may cleave double stranded and single stranded DNA. The term "DNases" and the expression "a polypeptide with DNase activity" are used interchangeably throughout the application. For purposes of the present invention, DNase activity is determined according to the procedure described in the Assay I.

Preferably the DNase is selected from any of the enzyme classes E.C.3.1, preferably E.C.3.1.21, e.g. such as E.C.3.1.21.X, where X = 1, 2, 3, 4, 5, 6, 7, 8 or 9, or e.g. Deoxyribonuclease I, Deoxyribonuclease IV, Type I site-specific deoxyribonuclease, Type II site-specific deoxyribonuclease, Type III site-specific deoxyribonuclease, CC-preferring endo-deoxyribonuclease, Deoxyribonuclease V, T(4) deoxyribonuclease II, T(4) deoxyribonuclease IV or E.C. 3.1.22.Y where Y = 1, 2, 4 or 5, e.g. Deoxyribonuclease II, Aspergillus deoxyribonuclease K(1), Crossover junction endo-deoxyribonuclease, Deoxyribonuclease X.

Preferably, the polypeptide having DNase activity is obtained from a microorganism and the DNase is a microbial enzyme. The DNase is preferably of fungal or bacterial origin.

The DNase may be obtainable from Bacillus e.g. *Bacillus, such* as a *Bacillus licheniformis, Bacillus subtilis, Bacillus sp-62451, Bacillus horikoshii, Bacillus sp-62451, Bacillus sp-16840, Bacillus sp-62668, Bacillus sp-13395, Bacillus horneckiae, Bacillus sp-11238, Bacillus cibi, Bacillus idriensis, Bacillus sp-62520, Bacillus sp-16840, Bacillus sp-62668, Bacillus algicola, Bacillus vietnamensis, Bacillus hwajinpoensis, Bacillus indicus, Bacillus marisflavi, Bacillus luciferensis, Bacillus sp. SA2-6.*

The DNase may also be obtained from any of the following *Pyrenochaetopsis sp. , Vibrissea flavovirens, Set-*

*osphaeria rostrate, Endophragmiella valdina, Corynespora cassiicola, Paraphoma sp. XZ1965, Monilinia fructicola, Curvularia lunata, Penicillium reticulisporum, Penicillium quercetorum, Setophaeosphaeria sp., Alternaria, Alternaria sp. XZ2545, Trichoderma reesei, Chaetomium thermophilum, Scytalidium thermophilum, Metapochonia suchlasporia, Daldinia fissa, Acremonium sp. XZ2007, Acremonium sp. XZ2414, Acremonium dichromosporum, Sarocladium sp. XZ2014, Metarhizium sp. HNA15-2, Isaria tenuipes Scytalidium circinatum, Metarhizium lepidiotae, Thermobispora bispora, Sporormia fimetaria, Pycnidiophora cf. dispera, Enviromental sample D, Enviromental sample O, Clavicipitaceae sp-70249, Westerdykella sp. AS85-2, Humicolopsis cephalosporioides, Neosartorya* massa, *Roussoella intermedia, Pleosporales, Phaeosphaeria or Didymosphaeria futilis.*

The DNases to be used in a composition of the invention preferable belong to the NUC1 group of DNases. The NUC1 group of DNases comprises polypeptides which in addition to having DNase activity, may comprise one or more of the motifs [T/D/S][G/N]PQL (SEQ ID NO 69), [F/L/Y/I]A[N/R]D[L/I/P/V] (SEQ ID NO: 70), or C[D/N]T[A/R] (SEQ ID NO: 71).

The DNases preferably comprise a NUC1_A domain [D/Q][I/V]DH (SEQ ID NO 72). In addition to comprising any of the domain motifs [T/D/S][G/N]PQL, [F/L/Y/I]A[N/R]D[L/I/P/V] or C[D/N]T[A/R] the polypeptides having DNase activity, to be used in a composition of the invention, may comprise the NUC1_A domain and may share the common motif [D/Q][I/V]DH (SEQ ID NO 72).

The DNases to be added to a composition of the invention preferably belong to the group of DNases comprised in the GYS-clade, which are group of DNases on the same branch of a phylogenetic tree having both structural and functional similarities. These NUC1 and/or NUC1_A DNases comprise the conservative motifs [D/M/L][S/T]GYSR[D/N] (SEQ ID NO: 73) or ASXNRSKG (SEQ ID NO: 74) and share similar structural and functional properties. The DNases of the GYS-clade are preferably obtained from *Bacillus* genus.

One embodiment of the invention relates to a composition comprising an RNase and a polypeptide of the GYS clade having DNase activity, wherein the polypeptide comprises one or both motifs [D/M/L][S/T]GYSR[D/N] (SEQ ID NO: 73), ASXNRSKG (SEQ ID NO: 74) and wherein the polypeptide has at least 80%, at least 85%, at least 90%, at least 95%, at least 98%, at least 99% or 100% sequence identity to the polypeptide shown in SEQ ID NO: 13.

Other DNases (not according to the invention) may be selected from the group consisting of:

a) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 1,
b) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 2,
c) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 3,
d) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 4,
e) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 5,
f) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 6,
g) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 7,
h) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 8,
i) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 9,
j) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 10,
k) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 11,
l) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 12,
m) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 14,
n) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 15,
o) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 16,
p) a polypeptide having at least 60%, at least 65%, at least 70%, at least 75% at least 80%, at least 85%, at least

90%, at least 95%, at least 98%, at least 99% or 100% sequence identity to the polypeptide shown in SEQ ID NO: 17,
q) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 18,
r) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 19,
s) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 20,
t) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 21,
u) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 22,
v) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 23,
w) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 24, and
x) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 25.

Polypeptides having DNase activity and which comprise the GYS-clade motifs have shown particularly good deep cleaning properties, e.g. the DNases are particularly effective in removing or reducing components of organic matter, such as biofilm, from an item such as a textile or a hard surface. In addition, these DNases are particularly effective in removing or reducing malodor, from an item such as a textile or a hard surface. Further, the GYS-clade DNases are particularly effective in preventing redeposition when laundering an item such as textile.

Other DNases (not according to the invention) may belong to the group of DNases comprised in the NAWK-clade, which are NUC1 and NUC1_A DNases, which may further comprise the conservative motifs [V/I]PL[S/A]NAWK (SEQ ID NO: 75) or NPQL (SEQ ID NO: 76).

Such polypeptides of the NAWK-clade having DNase activity, optionally comprising one or both motifs [V/I]PL[S/A]NAWK (SEQ ID NO: 75) or NPQL (SEQ ID NO: 76), may be selected from the group consisting of:

a) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 26,
b) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 27,
c) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 28,
d) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 29,
e) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 30,
f) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 31,
g) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 32,
h) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 33,
i) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 34,
j) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 35,
k) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 36,
l) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 37, and
m) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 38.

Polypeptides having DNase activity and which comprise the NAWK-clade motifs have shown particularly good deep cleaning properties e.g. the DNases are particularly effective in removing or reducing components of organic matter, such as biofilm, from an item such as a textile or a hard surface. In addition, these DNases are particularly effective in removing or reducing malodor, from an item such as a textile or a hard surface. Further, the NAWK-clade DNases are particularly effective in preventing redeposition when laundering an item such as textile.

Other DNases (not according to the invention) which may be added to a composition belong to the group of DNases comprised in the KNAW-clade, which are NUC1 and NUC1_A DNases which may further comprise the conservative motifs P[Q/E]L[W/Y] (SEQ ID NO: 77) or [K/H/E]NAW (SEQ ID NO: 78).

Such polypeptides of the KNAW clade having DNase activity, optionally comprising one or both motifs P[Q/E]L[W/Y] (SEQ ID NO: 77) or [K/H/E]NAW (SEQ ID NO: 78), may be selected from the group consisting of:

a) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 39,
b) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 40,
c) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 41,
d) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 42,
e) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 43
f) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 44,
g) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 45,
h) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 46,
i) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 47,
j) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 48,
k) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 49,
l) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 50, and
m) a polypeptide having at least 60%, at least 65%, at least 70%, at least 75% at least 80%, at least 85%, at least 90%, at least 95%, at least 98%, at least 99% or 100% sequence identity to the polypeptide shown in SEQ ID NO: 51.

Polypeptides having DNase activity and which comprise the KNAW-clade motifs have shown particularly good deep cleaning properties e.g. the DNases are particularly effective in removing or reducing components of organic matter, such as biofilm, from an item such as a textile or a hard surface. In addition, these DNases are particularly effective in removing or reducing malodor, from an item such as a textile or a hard surface. Further, the KNAW-clade DNases are particularly effective in preventing redeposition when laundering an item such as textile.

Compositions comprising an RNase may comprise a polypeptide obtainable from *Bacillus* e.g. obtainable from *Bacillus cibi* and having a sequence identity to the polypeptide shown in SEQ ID NO: 13 of at least 80%, at least 85%, at least 90%, at least 95%, at least 96%, at least 97%, at least 98%, at least 99%, or 100% and which has DNase activity. In one aspect, the polypeptide differs by up to 10 amino acids, e.g., 1, 2, 3, 4, 5, 6, 7, 8, 9, or 10, from the mature polypeptide shown in SEQ ID NO: 13.

Polypeptides having RNase activity (RNase)

The term "RNase" is an abbreviation of the term ribonuclease, which means a nuclease having RNase activity (EC 3.1.2.7) that catalyzes the degradation of RNA into smaller components. Ribonucleases can be divided into endoribonucleases and exoribonucleases. In one embodiment, the present invention relates to e.g. endoribonucleases. For purposes of the present invention, RNase activity is determined according to the procedure described in Assay II. The RNase to be incorporated in a composition of the invention includes RNases from the PF00545 family of RNases.

RNases to be added to a composition may be selected from RNases with E.C.3.1.26.X, where X = 1, 2, 3, 4,

5, 6, 7, 8, 9, 10, 11, 12 and 13 e.g. the RNases *Physarum polycephalum* RNase, RNase alpha, RNase III, RNase C, RNase H, RNase HII, RNase P, RNase IV, RNase P4, RNase M5, RNase poly-U specific, RNase IX, RNase Z, RNase E, RNase L or Retroviral RNase H. The RNase may also be selected from EC.3.1.27. Y, where Y= 1, 2, 3, 4, 5, 6, 7, 8, 9, 10 e.g. the RNases; RNase T(2), *Bacillus subtilis* RNase, RNase T(1), RNase U(2), Pancreatic RNase, RNase A, RNase I, Enterobacter RNase, RNase F, RNase V, rRNA endonuclease and e.g. RNase B Glycoprotein standard from bovine pancreas.

Other RNases are RNase A (Bovine pancreas) UniProt - P61823, RNase HI (E. coli) Uniprot- P0A7Y4, RNase HII (E. coli) UniProt - P10442, RNase III (E. coli) UniProt - P0A7Y0, RNase T1 (A. oryzae) UniProt - P00651, RNase T2 (A. oryzae) Uniprot - P10281 or closely related homologues e.g. RNase having at least 80%, or at least 85% or at least 90% or at least 95% or at least 98% sequence identity hereto.

The RNase may be obtainable from *Paenibacillus* e.g. *Paenibacillus sp-18057, Paenibacillus sp-62770, Paenibacillus sp-18006, Paenibacillus sp-62724, Paenibacillus tundrae.* Alternatively, the RNase may be obtained from *Amycolatopsis azurea, Acremonium alcalophilum, Erwinia persicina, Saccharothrix sp-62935, Saccharopolyspora endophytica, Amycolatopsis circi, Alkalimonas sp-62516, Nonomuraea dietziae.*

An RNase may comprise any of the following or all of the motifs EYTV (SEQ ID NO 82), [YRF]E[AYFWC]D (SEQ ID NO 83), IGGD (SEQ ID NO 84), YPH, HTGA (SEQ ID NO 85) or DRV.

One embodiment of the invention relates to a composition comprising a polypeptide having RNase activity, optionally wherein the polypeptide comprises one or all the motifs EYTV (SEQ ID NO 82), [YRF]E[AYFWC]D (SEQ ID NO 83), IGGD (SEQ ID NO 84), YPH, HTGA (SEQ ID NO 85) or DRV and wherein the polypeptide is selected from the group of polypeptides:

a) a polypeptide having at least 80%, at least 85%, at least 90%, at least 95%, at least 98%, at least 99% or 100% sequence identity to the polypeptide shown in SEQ ID NO: 87,
b) a polypeptide having at least 80%, at least 85%, at least 90%, at least 95%, at least 98%, at least 99% or 100% sequence identity to the polypeptide shown in SEQ ID NO: 90,
c) a polypeptide having at least 80%, at least 85%, at least 90%, at least 95%, at least 98%, at least 99% or 100% sequence identity to the polypeptide shown in SEQ ID NO: 95,
d) a polypeptide having at least 80%, at least 85%, at least 90%, at least 95%, at least 98%, at least 99% or 100% sequence identity to the polypeptide shown in SEQ ID NO: 96,
e) a polypeptide having at least 80%, at least 85%, at least 90%, at least 95%, at least 98%, at least 99% or 100% sequence identity to the polypeptide shown in SEQ ID NO: 97,
f) a polypeptide having at least 80%, at least 85%, at least 90%, at least 95%, at least 98%, at least 99% or 100% sequence identity to the polypeptide shown in SEQ ID NO: 98,
g) a polypeptide having at least 80%, at least 85%, at least 90%, at least 95%, at least 98%, at least 99% or 100% sequence identity to the polypeptide shown in SEQ ID NO: 99,
h) a polypeptide having at least 80%, at least 85%, at least 90%, at least 95%, at least 98%, at least 99% or 100% sequence identity to the polypeptide shown in SEQ ID NO: 100,
i) a polypeptide having at least 80%, at least 85%, at least 90%, at least 95%, at least 98%, at least 99% or 100% sequence identity to the polypeptide shown in SEQ ID NO: 101,
j) a polypeptide having at least 80%, at least 85%, at least 90%, at least 95%, at least 98%, at least 99% or 100% sequence identity to the polypeptide shown in SEQ ID NO: 102,
k) a polypeptide having at least 80%, at least 85%, at least 90%, at least 95%, at least 98%, at least 99% or 100% sequence identity to the polypeptide shown in SEQ ID NO: 103, and
l) a polypeptide having at least 80%, at least 85%, at least 90%, at least 95%, at least 98%, at least 99% or 100% sequence identity to the polypeptide shown in SEQ ID NO: 104.

In some preferred embodiments of the invention a DNase of the invention is combined with an RNase wherein the RNase is any of the following:
In some embodiments, the present invention relates to compositions comprising a polypeptide obtainable from *Paenibacillus sp-62770* and having a sequence identity to the polypeptide shown in SEQ ID NO: 87 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 87.

In some embodiments, the present invention relates to compositions comprising a polypeptide obtainable from *Acremonium alcalophilum* and having a sequence identity to the polypeptide shown in SEQ ID NO: 90 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 90.

In some embodiments, the present invention relates to compositions comprising a polypeptide obtainable from *Saccharothrix sp-62935* and having a sequence identity to the polypeptide shown in SEQ ID NO: 95 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 95.

In some embodiments, the present invention relates to compositions comprising a polypeptide obtainable from *Saccharopolyspora endophytica* and having a sequence identity to the polypeptide shown in SEQ ID NO: 96 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 96.

In some embodiments, the present invention relates to compositions comprising a polypeptide obtainable from *Amycolatopsis circi* and having a sequence identity to the polypeptide shown in SEQ ID NO: 97 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 97.

In some embodiments, the present invention relates to compositions comprising a polypeptide obtainable from *Paenibacillus sp-62770* and having a sequence identity to the polypeptide shown in SEQ ID NO: 98 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 98.

In some embodiments, the present invention relates to compositions comprising a polypeptide obtainable from *Paenibacillus sp-18006* and having a sequence identity to the polypeptide shown in SEQ ID NO: 99 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 99.

In some embodiments, the present invention relates to compositions comprising a polypeptide obtainable from *Paenibacillus sp-62724* and having a sequence identity to the polypeptide shown in SEQ ID NO: 100 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 100.

In some embodiments, the present invention relates to compositions comprising a polypeptide obtainable from *Alkalimonas sp-62516* and having a sequence identity to the polypeptide shown in SEQ ID NO: 101 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 101.

In some embodiments, the present invention relates to compositions comprising a polypeptide obtainable from *Nonomuraea dietziae* and having a sequence identity to the polypeptide shown in SEQ ID NO: 102 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 102.

In some embodiments, the present invention relates to compositions comprising a polypeptide obtainable from *Trichoderma harzianum* and having a sequence identity to the polypeptide shown in SEQ ID NO: 103 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 103.

In some embodiments, the present invention relates to compositions comprising a polypeptide obtainable from *Fusarium solani* and having a sequence identity to the polypeptide shown in SEQ ID NO: 104 of at least 60%, e.g., at least 65%, at least 70%, at least 75%, at least 80%, at least 85%, at least 90%, at least 95%, at least 96%, at least 97%, at least 98%, at least 99%, or 100% and which have RNase activity. In one aspect, the polypeptides differ by up to 10 amino acids, e.g., 1, 2, 3, 4, 5, 6, 7, 8, 9, or 10, from the mature polypeptide shown in SEQ ID NO: 104.

**Composition**

The invention relates to cleaning e.g. detergent compositions comprising an enzyme combination of the present invention in combination with one or more additional cleaning composition components. The choice of additional components is within the skill of the artisan and includes conventional ingredients, including the exemplary non-limiting components set forth below. An enzyme blend of the current invention comprises a DNase and an RNase. One embodiment of the invention relates to a cleaning composition comprising a DNase, an RNase and a cleaning component.

The DNase is preferably microbial, preferably obtained from bacteria or fungi.

The DNase may be obtained from bacteria. The DNase may be obtained from *Bacillus,* preferably *Bacillus cibi, Bacillus horikoshii, Bacillus licheniformis, Bacillus subtilis, Bacillus horneckiae, Bacillus idriensis, Bacillus algicola, Bacillus vietnamensis, Bacillus hwajinpoensis, Bacillus indicus, Bacillus marisflavi or Bacillus luciferensis.*

The RNase may be obtained from the genus *Paenibacillus* e.g. *Paenibacillus sp-18057, Paenibacillus sp-62770, Paenibacillus sp-18006, Paenibacillus sp-62724, Paenibacillus tundrae.* Alternatively, the RNase may be obtained from *Amycolatopsis azurea, Acremonium alcalophilum, Erwinia persicina, Saccharothrix sp-62935, Saccharopolyspora endophytica, Amycolatopsis circi, Alkalimonas sp-62516, Nonomuraea dietziae, Trichoderma harzianum* or *Fusarium solani.* A cleaning composition may comprise a DNase, an RNase and a cleaning component, wherein the DNase is obtained from *Bacillus,* preferably *Bacillus cibi, Bacillus horikoshii, Bacillus licheniformis, Bacillus subtilis, Bacillus horneckiae, Bacillus idriensis, Bacillus algicola, Bacillus vietnamensis, Bacillus hwajinpoensis, Bacillus indicus, Bacillus marisflavi or Bacillus luciferensis* and wherein the RNase is selected from RNases obtained from *Paenibacillus e.g. Paenibacillus sp-18057, Paenibacillus sp-62770, Paenibacillus sp-18006, Paenibacillus sp-62724* and *Amycolatopsis azurea, Acremonium alcalophilum, Erwinia persicina, Saccharothrix sp-62935, Saccharopolyspora endophytica, Amycolatopsis circi, Alkalimonas sp-62516, Nonomuraea dietziae, Trichoderma harzianum* and *Fusarium solani.* A cleaning composition may comprise a DNase, an RNase and a cleaning component, wherein the DNase is obtained from *Bacillus,* preferably *Bacillus cibi, Bacillus horikoshii, Bacillus licheniformis, Bacillus subtilis, Bacillus horneckiae, Bacillus idriensis, Bacillus algicola, Bacillus vietnamensis, Bacillus hwajinpoensis, Bacillus indicus, Bacillus marisflavi or Bacillus luciferensis* and wherein the RNase is selected from;

a) a polypeptide having at least 80%, at least 85%, at least 90%, at least 95%, at least 98%, at least 99% or 100% sequence identity to the polypeptide shown in SEQ ID NO: 87,
b) a polypeptide having at least 80%, at least 85%, at least 90%, at least 95%, at least 98%, at least 99% or 100% sequence identity to the polypeptide shown in SEQ ID NO: 90;
c) a polypeptide having at least 80%, at least 85%, at least 90%, at least 95%, at least 98%, at least 99% or 100% sequence identity to the polypeptide shown in SEQ ID NO: 95;
d) a polypeptide having at least 80%, at least 85%, at least 90%, at least 95%, at least 98%, at least 99% or 100% sequence identity to the polypeptide shown in SEQ ID NO: 96;
e) a polypeptide having at least 80%, at least 85%, at least 90%, at least 95%, at least 98%, at least 99% or 100% sequence identity to the polypeptide shown in SEQ ID NO: 97;
f) a polypeptide having at least 80%, at least 85%, at least 90%, at least 95%, at least 98%, at least 99% or 100% sequence identity to the polypeptide shown in SEQ ID NO: 98;
g) a polypeptide having at least 80%, at least 85%, at least 90%, at least 95%, at least 98%, at least 99% or 100% sequence identity to the polypeptide shown in SEQ ID NO: 99;
h) a polypeptide having at least 80%, at least 85%, at least 90%, at least 95%, at least 98%, at least 99% or 100% sequence identity to the polypeptide shown in SEQ ID NO: 100;
i) a polypeptide having at least 80%, at least 85%, at least 90%, at least 95%, at least 98%, at least 99% or 100% sequence identity to the polypeptide shown in SEQ ID NO: 101;
j) a polypeptide having at least 80%, at least 85%, at least 90%, at least 95%, at least 98%, at least 99% or 100% sequence identity to the polypeptide shown in SEQ ID NO: 102;
k) a polypeptide having at least 80%, at least 85%, at least 90%, at least 95%, at least 98%, at least 99% or 100% sequence identity to the polypeptide shown in SEQ ID NO: 103; and
l) a polypeptide having at least 80%, at least 85%, at least 90%, at least 95%, at least 98%, at least 99% or 100% sequence identity to the polypeptide shown in SEQ ID NO: 104.

The DNases preferable belong to the NUC1 group of DNases and comprise one or more of the motifs [T/D/S][G/N]PQL (SEQ ID NO 69), [F/L/Y/I]A[N/R]D[L/I/P/V] (SEQ ID NO: 70), or C[D/N]T[A/R] (SEQ ID NO: 71). The DNases even more preferably comprise a NUC1_A domain [D/Q][I/V]DH (SEQ ID NO 72). In addition, the DNases may comprise any of the motifs [T/D/S][G/N]PQL, [F/L/Y/I]A[N/R]D[L/I/P/V] or C[D/N]T[A/R]. The DNases to be added to a composition of the invention preferably belong to the group of DNases comprised in the GYS-clade, which are group of DNases on the same branch of a phylogenetic tree having both structural and functional similarities. These NUC1 and/or NUC1_A DNases comprise the conservative motifs [D/M/L][S/T]GYSR[D/N] (SEQ ID NO: 73) or ASXNRSKG (SEQ ID NO: 74) and share similar structural and functional properties. The DNases of the GYS-clade are preferably obtained from *Bacillus* genus. A cleaning composition may comprise a DNase, an RNase and a cleaning component, wherein the DNase comprises one or both motif(s) [D/M/L][S/T]GYSR[D/N] (SEQ ID NO: 73) or ASXNRSKG (SEQ ID NO: 74). One embodiment of the invention relates to a cleaning composition comprising a DNase, an RNase and a cleaning component, wherein the DNase comprises one or both motif(s) [D/M/L][S/T]GYSR[D/N] (SEQ ID NO: 73) or ASXNRSKG (SEQ ID NO: 74), wherein the RNase is selected from;

a) a polypeptide having at least 80%, at least 85%, at least 90%, at least 95%, at least 98%, at least 99% or 100% sequence identity to the polypeptide shown in SEQ ID NO: 87,
b) a polypeptide having at least 80%, at least 85%, at least 90%, at least 95%, at least 98%, at least 99% or 100% sequence identity to the polypeptide shown in SEQ ID NO: 90;
c) a polypeptide having at least 80%, at least 85%, at least 90%, at least 95%, at least 98%, at least 99% or 100% sequence identity to the polypeptide shown in SEQ ID NO: 95;
d) a polypeptide having at least 80%, at least 85%, at least 90%, at least 95%, at least 98%, at least 99% or 100% sequence identity to the polypeptide shown in SEQ ID NO: 96;
e) a polypeptide having at least 80%, at least 85%, at least 90%, at least 95%, at least 98%, at least 99% or 100% sequence identity to the polypeptide shown in SEQ ID NO: 97;
f) a polypeptide having at least 80%, at least 85%, at least 90%, at least 95%, at least 98%, at least 99% or 100% sequence identity to the polypeptide shown in SEQ ID NO: 98;
g) a polypeptide having at least 80%, at least 85%, at least 90%, at least 95%, at least 98%, at least 99% or 100% sequence identity to the polypeptide shown in SEQ ID NO: 99;
h) a polypeptide having at least 80%, at least 85%, at least 90%, at least 95%, at least 98%, at least 99% or 100% sequence identity to the polypeptide shown in SEQ ID NO: 100;
i) a polypeptide having at least 80%, at least 85%, at least 90%, at least 95%, at least 98%, at least 99% or 100% sequence identity to the polypeptide shown in SEQ ID NO: 101;
j) a polypeptide having at least 80%, at least 85%, at least 90%, at least 95%, at least 98%, at least 99% or 100% sequence identity to the polypeptide shown in SEQ ID NO: 102;
k) a polypeptide having least 60%, at least 65%, at least 70%, at least 75%, at least 80%, at least 85%, at least 90%, at least 95%, at least 98%, at least 99% or 100% sequence identity to the polypeptide shown in SEQ ID NO: 103; and
l) a polypeptide having at least 80%, at least 85%, at least 90%, at least 95%, at least 98%, at least 99% or 100% sequence identity to the polypeptide shown in SEQ ID NO: 104.

The RNases preferably comprise one or more of the motif(s) EYTV (SEQ ID NO 82), [YRF]E[AYFWC]D (SEQ ID NO 83), IGGD (SEQ ID NO 84), YPH, HTGA (SEQ ID NO 85) or DRV.

A cleaning composition may comprise a DNase, an RNase and a cleaning component, wherein the RNase comprise one or more of the motif(s) EYTV (SEQ ID NO 82), [YRF]E[AYFWC]D (SEQ ID NO 83), IGGD (SEQ ID NO 84), YPH, HTGA (SEQ ID NO 85), DRV and wherein the DNase one or both of the motifs [D/M/L][S/T]GYSR[D/N] (SEQ ID NO: 73), ASXNRSKG (SEQ ID NO: 74) and wherein the DNase is a polypeptide having at least 80%, at least 85%, at least 90%, at least 95%, at least 98%, at least 99% or 100% sequence identity to the polypeptide shown in SEQ ID NO: 13.

The DNase is preferably a bacillus DNase, such as a *Bacillus cibi, Bacillus subtilis* or *Bacillus licheniformis.*

One embodiment of the invention relates to a cleaning composition comprising a DNase, an RNase and a cleaning component, wherein the DNase has at least 80%, at least 85%, at least 90%, at least 95%, at least 98%, at least 99% or 100% sequence identity to the amino acid sequence shown in SEQ ID NO 13 and wherein the RNase is selected from the group consisting of RNases comprising an amino acid sequence with;

i) at least 80%, at least 85%, at least 90%, at least 95%, at least 98%, at least 99% or 100% sequence identity to the polypeptide shown in SEQ ID NO: 87,
ii) at least 80%, at least 85%, at least 90%, at least 95%, at least 98%, at least 99% or 100% sequence identity to the polypeptide shown in SEQ ID NO: 90,
iii) at least 80%, at least 85%, at least 90%, at least 95%, at least 98%, at least 99% or 100% sequence identity to the polypeptide shown in SEQ ID NO: 95,
iv) at least 80%, at least 85%, at least 90%, at least 95%, at least 98%, at least 99% or 100% sequence identity to the polypeptide shown in SEQ ID NO: 96,
v) at least 80%, at least 85%, at least 90%, at least 95%, at least 98%, at least 99% or 100% sequence identity to the polypeptide shown in SEQ ID NO: 97,
vi) at least 80%, at least 85%, at least 90%, at least 95%, at least 98%, at least 99% or 100% sequence identity to the polypeptide shown in SEQ ID NO: 98,
vii) at least 80%, at least 85%, at least 90%, at least 95%, at least 98%, at least 99% or 100% sequence identity to the polypeptide shown in SEQ ID NO: 99,
viii) at least 80%, at least 85%, at least 90%, at least 95%, at least 98%, at least 99% or 100% sequence identity to the polypeptide shown in SEQ ID NO: 100,
ix) at least 80%, at least 85%, at least 90%, at least 95%, at least 98%, at least 99% or 100% sequence identity to the polypeptide shown in SEQ ID NO: 101,

x) at least 80%, at least 85%, at least 90%, at least 95%, at least 98%, at least 99% or 100% sequence identity to the polypeptide shown in SEQ ID NO: 102,
xi) at least 80%, at least 85%, at least 90%, at least 95%, at least 98%, at least 99% or 100% sequence identity to the polypeptide shown in SEQ ID NO: 103, and
xii) at least 80%, at least 85%, at least 90%, at least 95%, at least 98%, at least 99% or 100% sequence identity to the polypeptide shown in SEQ ID NO: 104.

The invention relates to cleaning compositions comprising an enzyme combination of the present invention in combination with one or more additional cleaning composition components. The choice of additional components is within the skill of the artisan and includes conventional ingredients, including the exemplary non-limiting components set forth below.

One embodiment if the invention relates to a composition comprising;

a) at least 0.001 ppm of at least one DNase, wherein the DNase:

i) comprises one or both motifs [D/M/L][S/T]GYSR[D/N] (SEQ ID NO: 73) or ASXNRSKG (SEQ ID NO: 74); and
ii) is a polypeptide having at least 80%, at least 85%, at least 90%, at least 95%, at least 98%, at least 99% or 100% sequence identity to the polypeptide shown in SEQ ID NO: 13, and

b) at least 0.001 ppm of one or more RNase, wherein the RNase:

i) is a polypeptide having at least 80%, at least 85%, at least 90%, at least 95%, at least 98%, at least 99% or 100% sequence identity to the polypeptide shown in SEQ ID NO: 87,
ii) a polypeptide having at least 80%, at least 85%, at least 90%, at least 95%, at least 98%, at least 99% or 100% sequence identity to the polypeptide shown in SEQ ID NO: 90,
iii) a polypeptide having at least 80%, at least 85%, at least 90%, at least 95%, at least 98%, at least 99% or 100% sequence identity to the polypeptide shown in SEQ ID NO: 95,
iv) a polypeptide having at least 80%, at least 85%, at least 90%, at least 95%, at least 98%, at least 99% or 100% sequence identity to the polypeptide shown in SEQ ID NO: 96,
v) a polypeptide having at least 80%, at least 85%, at least 90%, at least 95%, at least 98%, at least 99% or 100% sequence identity to the polypeptide shown in SEQ ID NO: 97,
vi) a polypeptide having at least 80%, at least 85%, at least 90%, at least 95%, at least 98%, at least 99% or 100% sequence identity to the polypeptide shown in SEQ ID NO: 98,
vii) a polypeptide having at least 80%, at least 85%, at least 90%, at least 95%, at least 98%, at least 99% or 100% sequence identity to the polypeptide shown in SEQ ID NO: 99,
viii) a polypeptide having at least 80%, at least 85%, at least 90%, at least 95%, at least 98%, at least 99% or 100% sequence identity to the polypeptide shown in SEQ ID NO: 100,
ix) a polypeptide having at least 80%, at least 85%, at least 90%, at least 95%, at least 98%, at least 99% or 100% sequence identity to the polypeptide shown in SEQ ID NO: 101,
x) a polypeptide having at least 80%, at least 85%, at least 90%, at least 95%, at least 98%, at least 99% or 100% sequence identity to the polypeptide shown in SEQ ID NO: 102,
xi) a polypeptide having at least 80%, at least 85%, at least 90%, at least 95%, at least 98%, at least 99% or 100% sequence identity to the polypeptide shown in SEQ ID NO: 103, and
xii) a polypeptide having at least 80%, at least 85%, at least 90%, at least 95%, at least 98%, at least 99% or 100% sequence identity to the polypeptide shown in SEQ ID NO: 104; and

c) At least one cleaning component, preferably selected from surfactants, builders, bleach components, polymers and dispersing agents.

Optionally the cleaning composition comprises at least 0.001 ppm of one or more protease, selected from,

i) a protease variant of a protease parent, wherein the protease variant comprises one or more alteration(s) compared to a protease shown in SEQ ID NO 79 or SEQ ID NO 80 in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269, wherein the positions correspond to the positions of the protease shown in SEQ ID NO 79 and wherein the protease variant has at least 80% sequence identity to SEQ ID NO 79, SEQ ID NO 80 or SEQ ID NO 81;

ii) a protease variant of a protease parent, wherein the protease variant comprises one or more mutation selected from the group consisting of S3T, V4I, S9R, S9E, A15T, S24G, S24R, K27R, N42R, S55P, G59E, G59D, N60D, N60E, V66A, N74D, N85S, N85R, G96S, G96A, S97G, S97D, S97A, S97SD, S99E, S99D, S99G, S99M, S99N, S99R, S99H, S101A, V102I, V102Y, V102N, S104A, G116V, G116R, H118D, H118N, N120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A and R269H, wherein the positions correspond to the positions of the protease shown in SEQ ID NO 79, wherein the protease variant has at least 80% sequence identity to SEQ ID NO 79, SEQ ID NO 80 or SEQ ID NO 81;

iii) a protease comprising a substitution at one or more positions corresponding to positions 171, 173, 175, 179, or 180 of SEQ ID NO: 81, compared to the protease shown in SEQ ID NO 81, wherein the protease variant has a sequence identity of at least 75% but less than 100% to amino acid 1 to 311 of SEQ ID NO 81,

a protease comprising the amino acid sequence shown in SEQ ID NO 79, 80 or 81 or a protease having at least 80% sequence identity to; the polypeptide comprising amino acids 1-269 of SEQ ID NO 79, the polypeptide comprising amino acids 1-311 of SEQ ID NO 81 or the polypeptide comprising amino acids 1-275 of SEQ ID NO 80.

The RNase and DNase may be included in the cleaning composition of the present invention at a level of from 0.01 to 1000 ppm, from 1 ppm to 1000 ppm, from 10 ppm to 1000 ppm, from 50 ppm to 1000 ppm, from 100 ppm to 1000 ppm, from 150 ppm to 1000 ppm, from 200 ppm to 1000 ppm, from 250 ppm to 1000 ppm, from 250 ppm to 750 ppm, from 250 ppm to 500 ppm. The DNases above may be combined with RNase to form a blend to be added to the wash liquor solution according to the invention. The concentration of the DNase in the wash liquor solution is typically in the range of wash liquor from 0.00001ppm to 10 ppm, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, 0.1 ppm to 10 ppm, from 0.2 ppm to 10 ppm, from 0.5 ppm to 5 ppm. The concentration of the RNase in the wash liquor solution is typically in the range of wash liquor from 0.00001ppm to 10 ppm, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, 0.1 ppm to 10 ppm, from 0.2ppm to 10 ppm, from 0.5 ppm to 5 ppm. The DNases may be combined with any of the RNases mentioned above to form a blend to be added to a composition according to the invention.

One embodiment relates to a cleaning composition comprising a DNase, an RNase and at least one cleaning component, wherein the amount of DNase in the composition is from 0.01 to 1000 ppm and the amount of RNase is from 0.01 to 1000 ppm.

One aspect relates to a method of formulating a cleaning composition a cleaning composition comprising a DNase, an RNase and at least one cleaning component, comprising adding a DNase, an RNase and at least one cleaning component.

The choice of cleaning components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

<u>Surfactants</u>

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and may include any conventional surfactant(s) known in the art.

When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES)

including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein the detergent will usually contain from about 1% to about 40% by weigh of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.

When included therein the detergent will usually contain from about 0.01% to about 10 % by weight of a semipolar surfactant. Non-limiting examples of semipolar surfactants include amine oxides (AO) such as alkyldimethylamineoxide, N-(coco alkyl)-N,N-dimethylamine oxide and N-(tallow-alkyl)-N,N-bis(2-hydroxyethyl)amine oxide and combinations thereof.

When included therein the detergent will usually contain from about 0.01% to about 10 % by weight of a zwitterionic surfactant. Non-limiting examples of zwitterionic surfactants include betaines such as alkyldimethylbetaines, sulfobetaines, and combinations thereof.

Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in cleaning detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)-aspartic acid (SMAS), N-(2-sulfoethyl)-aspartic acid (SEAS), N-(2-sulfomethyl)-glutamic acid (SMGL), N-(2-sulfoethyl)-glutamic acid (SEGL), N-methyliminodiacetic acid (MIDA), $\alpha$-alanine-N,N-diacetic acid ($\alpha$-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA) , taurine-N,N-diacetic acid (TUDA) and sulfomethyl-N,N-diacetic acid (SMDA), N-(2-hydroxyethyl)ethylenediamine-N,N',N"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

Bleaching Systems

The detergent may contain 0-30% by weight, such as about 1% to about 20%, of a bleaching system. Any bleaching system comprising components known in the art for use in cleaning detergents may be utilized. Suitable bleaching system components include sources of hydrogen peroxide; sources of peracids; and bleach catalysts or boosters.

Sources of hydrogen peroxide:

Suitable sources of hydrogen peroxide are inorganic persalts, including alkali metal salts such as sodium percarbonate and sodium perborates (usually mono- or tetrahydrate), and hydrogen peroxide-urea (1/1).

Sources of peracids:

Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone®) or alkaline earth-metal salts.

b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

Bleach catalysts and boosters

The bleaching system may also include a bleach catalyst or booster.

Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal compounds; such as iron or cobalt complexes.

In some embodiments, where a source of a peracid is included, an organic bleach catalyst or bleach booster may be used having one of the following formulae:

(i) 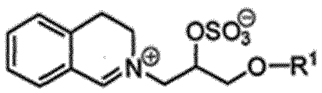

(ii) 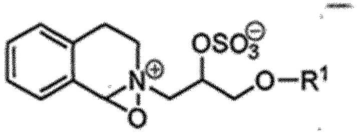

(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

## Metal care agents

Metal care agents may prevent or reduce the tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20- alkyl groups (e.g., C1-C20- alkyl groups) and hydroxyl, thio, phenyl or halogen such as fluorine, chlorine, bromine and iodine.

(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, K^TiF6 (e.g., K2TiF6), K^ZrF6 (e.g., K2ZrF6), CoSO4, Co(NOs)2 and Ce(NOs)3, zinc salts, for example zinc sulphate, hydrozincite or zinc acetate.;

(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859. Preferably the composition of the invention comprises from 0.1 to 5% by weight of the composition of a metal care agent, preferably the metal care agent is a zinc salt.

## Hydrotropes

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

## Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide anti redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of polyethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Suitable examples include PVP-K15, PVP-K30, ChromaBond S-400, ChromaBond S- 403E and Chromabond S-100 from Ashland Aqualon, and Sokalan® HP 165, Sokalan® HP 50 (Dispersing agent), Sokalan® HP 53 (Dispersing agent), Sokalan® HP 59 (Dispersing agent), Sokalan® HP 56 (dye transfer inhibitor), Sokalan® HP 66 K (dye transfer inhibitor) from BASF. Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated. Particularly preferred polymer is ethoxylated homopolymer Sokalan® HP 20 from BASF, which helps to prevent redeposition of soil in the wash liquor.

Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

Enzymes

The detergent additive as well as the detergent composition may comprise one or more additional enzymes such as one or more lipase, cutinase, an amylase, carbohydrase, cellulase, pectinase, mannanase, arabinase, galactanase, xylanase, oxidase, e.g., a laccase, and/or peroxidase.

In general, the properties of the selected enzyme(s) should be compatible with the selected detergent, (i.e., pH-optimum, compatibility with other enzymatic and non-enzymatic ingredients, etc.), and the enzyme(s) should be present in effective amounts.

**Proteases**

The term "protease" is defined herein as an enzyme that hydrolyses peptide bonds. It includes any enzyme belonging to the EC 3.4 enzyme group (including each of the thirteen subclasses thereof). The EC number refers to Enzyme Nomenclature 1992 from NC-IUBMB, Academic Press, San Diego, California, including supplements 1-5 published in Eur. J. Biochem. 1223: 1-5 (1994); Eur. J. Biochem. 232: 1-6 (1995); Eur. J. Biochem. 237: 1-5 (1996); Eur. J. Biochem. 250: 1-6 (1997); and Eur. J. Biochem. 264: 610-650 (1999); respectively. The most widely used proteases in the detergent industry such as laundry and dish wash are the serine proteases. Serine proteases is a subgroup of proteases characterised by having a serine in the active site, which forms a covalent adduct with the substrate. Serine proteases are characterized by having two active site amino acid residues apart from the serine, namely a histidine residue and an aspartic acid residue. Subtilase refer to a sub-group of serine protease according to Siezen et al., 1991, Protein Engng. 4: 719-737 and Siezen et al., 1997, Protein Science 6: 501-523. The subtilases may be divided into 6 sub-divisions, *i.e.,* the Subtilisin family, the Thermitase family, the Proteinase K family, the Lantibiotic peptidase family, the Kexin family and the Pyrolysin family. The term "protease activity" means a proteolytic activity (EC 3.4). Proteases usable in cleaning compositions of the present invention are mainly endopeptidases (EC 3.4.21). There are several protease activity types: The three main activity types are: trypsin-like where there is cleavage of amide substrates following Arg or Lys at P1, chymotrypsin-like where cleavage occurs following one of the hydrophobic amino acids at P1, and elastase-like with cleavage following an Ala at P1. For purposes of the present invention, protease activity is determined according to the Suc-AAPF-pNA activity assay.

Suitable proteases for the compositions of the invention include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

Examples of subtilases are those derived from *Bacillus* such as *Bacillus lentus, Bacillus alkalophilus, Bacillus subtilis, Bacillus amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147 and subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium protease* described in WO89/06270, WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially protease variants comprising a substitution in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269, wherein the positions correspond to the positions of the *Bacillus lentus* protease shown in SEQ ID NO 69. More preferred the protease variants may comprise one or more of the mutations selected from the group consisting of: S3T, V4I, S9R, S9E, A15T, S24G, S24R, K27R, N42R, S55P, G59E, G59D, N60D, N60E, V66A, N74D, N85S, N85R, G96S, G96A, S97G, S97D, S97A, S97SD, S99E, S99D, S99G, S99M, S99N, S99R, S99H, S101A, V102I, V102Y, V102N, S104A, G116V, G116R, H118D, H118N, N120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A and R269H. The protease variants are preferably variants of the Bacillus lentus protease (Savinase®) shown in SEQ ID NO 79 or the *Bacillus amyloliquefaciens* protease (BPN') shown in SEQ ID NO 80. The protease variants preferably have at least 80 % sequence identity to SEQ ID NO 79 or SEQ ID NO 80.

A protease variant comprising a substitution at one or more positions corresponding to positions 171, 173, 175, 179, or 180 of SEQ ID NO: 81, wherein said protease variant has a sequence identity of at least 75% but less than 100% to SEQ ID NO: 81.

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase® and Esperase® (Novozymes A/S), those sold under the tradename Maxatase®, Maxacai®, Maxapem®, Purafect Ox®, Purafect OxP®, Puramax®, FN2®, FN3®, FN4®, Excellase®, Excellenz P1000™, Excellenz P1250™, Eraser®, Preferenz P100™, Purafect Prime®, Preferenz P110™, Effectenz P1000™, Purafect®™, Effectenz P1050™, Purafect Ox®™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP *(Bacillus* alkalophilus subtilisin) from Kao.

**Cellulases**

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g., the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

**Mannanases**

Suitable mannanases include those of bacterial or fungal origin. Chemically or genetically modified mutants are included. The mannanase may be an alkaline mannanase of Family 5 or 26. It may be a wild-type from Bacillus or Humicola, particularly B. agaradhaerens, B. licheniformis, B. halodurans, B. clausii, or H. insolens. Suitable mannanases are described in WO 1999/064619. A commercially available mannanase is Mannaway (Novozymes A/S).

**Peroxidases/Oxidases**

Suitable peroxidases/oxidases include those of plant, bacterial or fungal origin. Chemically modified or protein engineered mutants are included. Examples of useful peroxidases include peroxidases from Coprinus, e.g., from C. cinereus, and variants thereof as those described in WO 93/24618, WO 95/10602, and WO 98/15257. Commercially available peroxidases include Guardzyme™ (Novozymes A/S).

**Lipases and Cutinases:**

Suitable lipases and cutinases include those of bacterial or fungal origin. Chemically modified or protein engineered mutant enzymes are included. Examples include lipase from Thermomyces, e.g. from T. lanuginosus (previously named Humicola lanuginosa) as described in EP258068 and EP305216, cutinase from Humicola, e.g. H. insolens (WO96/13580), lipase from strains of Pseudomonas (some of these now renamed to Burkholderia), e.g. P. alcaligenes or P. pseudoalcaligenes (EP218272), P. cepacia (EP331376), P. sp. strain SD705 (WO95/06720 & WO96/27002), P. wisconsinensis (WO96/12012), GDSL-type Streptomyces lipases (WO10/065455), cutinase from Magnaporthe grisea (WO10/107560), cutinase from Pseudomonas mendocina (US5,389,536), lipase from Thermobifida fusca (WO11/084412), Geobacillus stearothermophilus lipase (WO11/084417), lipase from Bacillus subtilis (WO11/084599), and lipase from Streptomyces griseus (WO11/150157) and S. pristinaespiralis (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to Candida antarctica lipase A (WO10/111143), acyltransferase from Mycobacterium smegmatis (WO05/56782), perhydrolases from the CE 7 family (WO09/67279), and variants of the M. smegmatis perhydrolase in particular the S54V variant used in the commercial product Gentle Power Bleach from Huntsman Textile Effects Pte Ltd (WO10/100028).

**Amylases:**

Suitable amylases include alpha-amylases and/or glucoamylases and may be of bacterial or fungal origin. Chemically modified or protein engineered mutants are included. Amylases include, for example, alpha-amylases obtained from Bacillus, e.g., a special strain of Bacillus licheniformis, described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase derived from B. amyloliquefaciens shown in SEQ ID NO: 6 of WO 2006/066594 and residues 36-483 of the B. licheniformis alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and

G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or

S125A+N128C+T131I+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:

E187P+I203Y+G476K
E187P+1203Y+R458N+T459S+D460T+G476K

wherein the variants optionally further comprise a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, I181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of I181 and/or G182. Most preferred amylase variants of SEQ ID NO: 1 are those having the substitutions:
N21D+D97N+V128I
wherein the variants optionally further comprise a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally

having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase, Preferenz S1000, PreferenzS100 and Preferenz S110 (from Genencor International Inc./DuPont).

**Peroxidases/Oxidases**

A peroxidase for use in the invention is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from Coprinopsis, e.g., from C. cinerea (EP 179,486), and variants thereof as those described in WO 93/24618, WO 95/10602, and WO 98/15257.

A suitable peroxidase includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions. Preferably, the haloperoxidase is a vanadium haloperoxidase, i.e., a vanadate-containing haloperoxidase. Haloperoxidases have been isolated from many different fungi, in particular from the fungus group dematiaceous hyphomycetes, such as Caldariomyces, e.g., C. fumago, Alternaria, Curvularia, e.g., C. verruculosa and C. inaequalis, Drechslera, Ulocladium and Botrytis.

Haloperoxidases have also been isolated from bacteria such as Pseudomonas, e.g., P. pyrrocinia and Streptomyces, e.g., S. aureofaciens.

A suitable oxidase includes in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5). Preferred laccase enzymes are enzymes of microbial origin. The enzymes may be derived from plants, bacteria or fungi (including filamentous fungi and yeasts). Suitable examples from fungi include a laccase derivable from a strain of Aspergillus, Neurospora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinopsis, e.g., C. cinerea, C. comatus, C. friesii, and C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata (WO 92/01046), or Coriolus, e.g., C. hirsutus (JP 2238885). Suitable examples from bacteria include a laccase derivable from a strain of Bacillus. A laccase derived from Coprinopsis or Myceliophthora is preferred; in particular, a laccase derived from Coprinopsis cinerea, as disclosed in WO 97/08325; or from Myceliophthora thermophila, as disclosed in WO 95/33836.

Dispersants

The cleaning compositions of the present invention can also contain dispersants. In particular, powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer inhibiting Agents

The cleaning compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent

The cleaning compositions of the present invention will preferably also contain additional components that may

tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(N-methyl-N-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2H-naphtho[1,2-d][1,2,3]triazol-2-yl)-2-[(E)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt % to upper levels of 0.5 or even 0.75 wt%.

Soil release polymers

The cleaning compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers is amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore, random graft co-polymers are suitable soil release polymers. Suitable graft co-polymers are described in more detail in WO 2007/138054, WO 2006/108856 and WO 2006/113314. Suitable polyethylene glycol polymers include random graft co-polymers comprising: (i) hydrophilic backbone comprising polyethylene glycol; and (ii) side chain(s) selected from the group consisting of: C4-C25 alkyl group, polypropylene, polybutylene, vinyl ester of a saturated C1-C6 mono-carboxylic acid, Cl-C 6 alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1: 1 to 1:5, or from 1: 1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents

The cleaning compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Rheology Modifiers

The cleaning compositions of the present invention may also include one or more rheology modifiers, structurants or thickeners, as distinct from viscosity reducing agents. The rheology modifiers are selected from the group

consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable cleaning composition components include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

Formulation of detergent products

The cleaning composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

Granular detergent formulations

Non-dusting granulates may be produced, e.g. as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The DNase and RNase may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme cogranulate for the detergent industry is disclosed in the IP.com disclosure IPCOM000200739D.

Another example of formulation of enzymes by the use of co-granulates are disclosed in WO 2013/188331, which relates to a detergent composition comprising (a) a multi-enzyme co- granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component. The multi-enzyme co-granule may comprise an enzyme of the invention and one or more enzymes

selected from the group consisting of proteases, lipases, cellulases, xyloglucanases, perhydrolases, peroxidases, lipoxygenases, laccases, hemicellulases, proteases, cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof. WO 2013/188331 also relates to a method of treating and/or cleaning a surface, preferably a fabric surface comprising the steps of (i) contacting said surface with the detergent composition as claimed and described herein in aqueous wash liquor, (ii) rinsing and/or drying the surface.

An embodiment of the invention relates to an enzyme granule/particle comprising the DNase and RNase. The granule is composed of a core, and optionally one or more coatings (outer layers) surrounding the core. Typically, the granule/particle size, measured as equivalent spherical diameter (volume based average particle size), of the granule is 20-2000 $\mu$m, particularly 50-1500 $\mu$m, 100-1500 $\mu$m or 250-1200 $\mu$m. The core may include additional materials such as fillers, fibre materials (cellulose or synthetic fibres), stabilizing agents, solubilising agents, suspension agents, viscosity regulating agents, light spheres, plasticizers, salts, lubricants and fragrances. The core may include binders, such as synthetic polymer, wax, fat, or carbohydrate. The core may comprise a salt of a multivalent cation, a reducing agent, an antioxidant, a peroxide decomposing catalyst and/or an acidic buffer component, typically as a homogenous blend. The core may consist of an inert particle with the enzyme absorbed into it, or applied onto the surface, e.g., by fluid bed coating. The core may have a diameter of 20-2000 $\mu$m, particularly 50-1500 $\mu$m, 100-1500 $\mu$m or 250-1200 $\mu$m. The core can be prepared by granulating a blend of the ingredients, e.g., by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Methods for preparing the core can be found in Handbook of Powder Technology; Particle size enlargement by C. E. Capes; Volume 1; 1980; Elsevier.

The core of the enzyme granule/particle may be surrounded by at least one coating, e.g., to improve the storage stability, to reduce dust formation during handling, or for colouring the granule. The optional coating(s) may include a salt coating, or other suitable coating materials, such as polyethylene glycol (PEG), methyl hydroxy-propyl cellulose (MHPC) and polyvinyl alcohol (PVA). Examples of enzyme granules with multiple coatings are shown in WO 93/07263 and WO 97/23606. The coating may be applied in an amount of at least 0.1% by weight of the core, e.g., at least 0.5%, 1% or 5%. The amount may be at most 100%, 70%, 50%, 40% or 30%. The coating is preferably at least 0.1 $\mu$m thick, particularly at least 0.5 $\mu$m, at least 1 $\mu$m or at least 5 $\mu$m. In a one embodiment, the thickness of the coating is below 100 $\mu$m. In another embodiment, the thickness of the coating is below 60 $\mu$m. In an even more particular embodiment the total thickness of the coating is below 40 $\mu$m. The coating should encapsulate the core unit by forming a substantially continuous layer. A substantially continuous layer is to be understood as a coating having few or no holes, so that the core unit it is encapsulating/enclosing has few or none uncoated areas. The layer or coating should be homogeneous in thickness. The coating can further contain other materials as known in the art, e.g., fillers, antisticking agents, pigments, dyes, plasticizers and/or binders, such as titanium dioxide, kaolin, calcium carbonate or talc. A salt coating may comprise at least 60% by weight w/w of a salt, *e.g.*, at least 65%, at least 70%, at least 75%, at least 80%, at least 85%, at least 90%, at least 95% or at least 99% by weight w/w. The salt may be added from a salt solution where the salt is completely dissolved or from a salt suspension wherein the fine particles is less than 50 $\mu$m, such as less than 10 $\mu$m or less than 5 $\mu$m. The salt coating may comprise a single salt or a mixture of two or more salts. The salt may be water soluble, and may have a solubility at least 0.1 grams in 100 g of water at 20°C, preferably at least 0.5 g per 100 g water, *e.g.*, at least 1 g per 100 g water, *e.g.,* at least 5 g per 100 g water. The salt may be an inorganic salt, *e.g.,* salts of sulfate, sulfite, phosphate, phosphonate, nitrate, chloride or carbonate or salts of simple organic acids (less than 10 carbon atoms, *e.g.*, 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used. The salt in the coating may have a constant humidity at 20°C above 60%, particularly above 70%, above 80% or above 85%, or it may be another hydrate form of such a salt (*e.g.*, anhydrate). The salt coating may be as described in WO 00/01793 or WO 2006/034710. Specific examples of suitable salts are NaCl ($CH_{20°C}$=76%), $Na_2CO_3$ ($CH_{20°C}$=92%), $NaNO_3$ ($CH_{20°C}$=73%), $Na_2HPO_4$ ($CH_{20°C}$=95%), $Na_3PO_4$ ($CH_{25°C}$=92%), $NH_4Cl$ ($CH_{20°C}$= 79.5%), $(NH_4)_2HPO_4$ ($CH_{20°C}$= 93,0%), $NH_4H_2PO_4$ ($CH_{20°C}$= 93.1%), $(NH_4)_2SO_4$ ($CH_{20°C}$=81.1%), KCl ($CH_{20°C}$=85%), $K_2HPO_4$ ($CH_{20°C}$=92%), $KH_2PO_4$ ($CH_{20°C}$=96.5%), $KNO_3$ ($CH_{20°C}$=93.5%), $Na_2SO_4$ ($CH_{20°C}$=93%), $K_2SO_4$ ($CH_{20°C}$=98%), $KHSO_4$ ($CH_{20°C}$=86%), $MgSO_4$ ($CH_{20°C}$=90%), $ZnSO_4$ ($CH_{20°C}$=90%) and sodium citrate ($CH_{25°C}$=86%). Other examples include $NaH_2PO_4$, $(NH_4)H_2PO_4$, $CuSO_4$, $Mg(NO_3)_2$ and magnesium acetate. The salt may be in anhydrous form, or it may be a hydrated salt, i.e. a crystalline salt hydrate with bound water(s) of crystallization, such as described in WO

99/32595. Specific examples include anhydrous sodium sulfate ($Na_2SO_4$), anhydrous magnesium sulfate ($MgSO_4$), magnesium sulfate heptahydrate ($MgSO_4 \cdot 7H_2O$), zinc sulfate heptahydrate ($ZnSO_4 \cdot 7H_2O$), sodium phosphate dibasic heptahydrate ($Na_2HPO_4 \cdot 7H_2O$), magnesium nitrate hexahydrate ($Mg(NO_3)_2(6H_2O)$), sodium citrate dihydrate and magnesium acetate tetrahydrate. Preferably the salt is applied as a solution of the salt, e.g., using a fluid bed.

One embodiment of the present invention provides a granule, which comprises:

(a) a core comprising a DNase and an RNase, and
(b) optionally a coating consisting of one or more layer(s) surrounding the core.

One embodiment of the invention relates to a granule, which comprises:

(a) a core comprising a DNase and an RNase wherein the RNase is selected from the group consisting of RNases comprising an amino acid sequence with;

i) at least 80%, at least 85%, at least 90%, at least 95%, at least 98%, at least 99% or 100% sequence identity to the polypeptide shown in SEQ ID NO: 87,
ii) at least 80%, at least 85%, at least 90%, at least 95%, at least 98%, at least 99% or 100% sequence identity to the polypeptide shown in SEQ ID NO: 90, and
iii) at least 80%, at least 85%, at least 90%, at least 95%, at least 98%, at least 99% or 100% sequence identity to the polypeptide shown in SEQ ID NO: 95,
iv) at least 80%, at least 85%, at least 90%, at least 95%, at least 98%, at least 99% or 100% sequence identity to the polypeptide shown in SEQ ID NO: 96,
v) at least 80%, at least 85%, at least 90%, at least 95%, at least 98%, at least 99% or 100% sequence identity to the polypeptide shown in SEQ ID NO: 97,
vi) at least 80%, at least 85%, at least 90%, at least 95%, at least 98%, at least 99% or 100% sequence identity to the polypeptide shown in SEQ ID NO: 98,
vii) at least 80%, at least 85%, at least 90%, at least 95%, at least 98%, at least 99% or 100% sequence identity to the polypeptide shown in SEQ ID NO: 99,
viii) at least 80%, at least 85%, at least 90%, at least 95%, at least 98%, at least 99% or 100% sequence identity to the polypeptide shown in SEQ ID NO: 100,
ix) at least 80%, at least 85%, at least 90%, at least 95%, at least 98%, at least 99% or 100% sequence identity to the polypeptide shown in SEQ ID NO: 101,
x) at least 80%, at least 85%, at least 90%, at least 95%, at least 98%, at least 99% or 100% sequence identity to the polypeptide shown in SEQ ID NO: 102,
xi) at least 80%, at least 85%, at least 90%, at least 95%, at least 98%, at least 99% or 100% sequence identity to the polypeptide shown in SEQ ID NO: 103,
xii) at least 80%, at least 85%, at least 90%, at least 95%, at least 98%, at least 99% or 100% sequence identity to the polypeptide shown in SEQ ID NO: 104,

and wherein the DNase has at least 80%, at least 85%, at least 90%, at least 95%, at least 98%, at least 99% or 100% sequence identity to the amino acid sequence shown in SEQ ID NO 13, and
(b) optionally a coating consisting of one or more layer(s) surrounding the core.

**Uses**

The present invention is also directed to methods for using the compositions thereof in laundry/textile/fabric cleaning (household laundry washing, industrial laundry washing) and hard surface cleaning (ADW, car wash, industrial surfaces).

The cleaning e.g. detergent composition of the present invention may be formulated, for example, as a hand or machine laundry composition including a laundry additive composition suitable for pre-treatment of stained fabrics and a rinse added fabric softener composition, or be formulated as a cleaning e.g. detergent composition for use in general household hard surface cleaning operations, or be formulated for hand or machine dishwashing operations. In a specific aspect, the present invention provides a detergent additive comprising one or more enzymes as described herein.

The compositions of the invention comprise a blend of DNase and RNase, and effectively reduce or remove organic components, such as RNA and DNA from surfaces such as textiles and hard surfaces e.g. dishes. One embodiment of the invention relates to the use of a cleaning composition comprising a DNase, an RNase and at least one cleaning component for reduction or removal of components of biofilm, such as DNA and RNase, of an item, wherein

the item is a textile or a hard surface.

One embodiment of the invention relates to the use of a cleaning composition comprising a DNase, at least one RNase and a cleaning component for deep cleaning of an item, wherein the item is a textile or a surface.

One embodiment of the invention relates to the use of a composition comprising a DNase and an RNase for reduction or removal of biofilm and/or compounds such as RNA and DNA of an item. One embodiment of the invention relates to the use of a cleaning composition comprising a DNase and an RNase for reduction or removal of biofilm and/or compounds such as RNA and DNA of an item such as textile. One embodiment of the invention relates to the use of a cleaning composition comprising a DNase and an RNase for deep cleaning when the cleaning composition is applied in e.g. laundry process.

One embodiment of the invention relates to the use of a composition comprising a DNase and RNase for reduction of redeposition or reduction of malodor. One embodiment of the invention relates to the use of a cleaning composition comprising a DNase and RNase for reduction of redeposition or reduction of malodor.

One embodiment of the invention relates to the use of a cleaning composition comprising a DNase and RNase for reduction of redeposition or reduction of malodor when the cleaning composition is applied in e.g. laundry process. One embodiment of the invention relates to the use of a cleaning composition comprising a DNase and RNase for reduction of redeposition or reduction of malodor on an item e.g. textile. In one embodiment, the composition is an anti-redeposition composition.

One embodiment of the invention relates to the use of a cleaning composition comprising a DNase and an RNase for deep cleaning of an item or reduction of redeposition or malodor, wherein the RNase is selected from the group consisting of polypeptides comprising;

i) a polypeptide having at least 80%, at least 85%, at least 90%, at least 95%, at least 98%, at least 99% or 100% sequence identity to the polypeptide shown in SEQ ID NO: 87,
ii) a polypeptide having at least 80%, at least 85%, at least 90%, at least 95%, at least 98%, at least 99% or 100% sequence identity to the polypeptide shown in SEQ ID NO: 90,
iii) a polypeptide having at least 80%, at least 85%, at least 90%, at least 95%, at least 98%, at least 99% or 100% sequence identity to the polypeptide shown in SEQ ID NO: 95,
iv) a polypeptide having at least 80%, at least 85%, at least 90%, at least 95%, at least 98%, at least 99% or 100% sequence identity to the polypeptide shown in SEQ ID NO: 96,
v) a polypeptide having at least 80%, at least 85%, at least 90%, at least 95%, at least 98%, at least 99% or 100% sequence identity to the polypeptide shown in SEQ ID NO: 97,
vi) a polypeptide having at least 80%, at least 85%, at least 90%, at least 95%, at least 98%, at least 99% or 100% sequence identity to the polypeptide shown in SEQ ID NO: 98,
vii) a polypeptide having at least 80%, at least 85%, at least 90%, at least 95%, at least 98%, at least 99% or 100% sequence identity to the polypeptide shown in SEQ ID NO: 99,
viii) a polypeptide having at least 80%, at least 85%, at least 90%, at least 95%, at least 98%, at least 99% or 100% sequence identity to the polypeptide shown in SEQ ID NO: 100,
ix) a polypeptide having at least 80%, at least 85%, at least 90%, at least 95%, at least 98%, at least 99% or 100% sequence identity to the polypeptide shown in SEQ ID NO: 101,
x) a polypeptide having at least 80%, at least 85%, at least 90%, at least 95%, at least 98%, at least 99% or 100% sequence identity to the polypeptide shown in SEQ ID NO: 102,
xi) a polypeptide having at least 80%, at least 85%, at least 90%, at least 95%, at least 98%, at least 99% or 100% sequence identity to the polypeptide shown in SEQ ID NO: 103, and
xii) a polypeptide having at least 80%, at least 85%, at least 90%, at least 95%, at least 98%, at least 99% or 100% sequence identity to the polypeptide shown in SEQ ID NO: 104.

One embodiment of the invention relates to the use of a cleaning composition comprising a DNase and an RNase for deep cleaning of an item or reduction of redeposition or malodor, wherein the RNase is selected from the group consisting of polypeptides comprising;

i) a polypeptide having at least 80%, at least 85%, at least 90%, at least 95%, at least 98%, at least 99% or 100% sequence identity to the polypeptide shown in SEQ ID NO: 87,
ii) a polypeptide having at least 80%, at least 85%, at least 90%, at least 95%, at least 98%, at least 99% or 100% sequence identity to the polypeptide shown in SEQ ID NO: 90,
iii) a polypeptide having at least 80%, at least 85%, at least 90%, at least 95%, at least 98%, at least 99% or 100% sequence identity to the polypeptide shown in SEQ ID NO: 95,
iv) a polypeptide having at least 80%, at least 85%, at least 90%, at least 95%, at least 98%, at least 99% or 100% sequence identity to the polypeptide shown in SEQ ID NO: 96,

v) a polypeptide having at least 80%, at least 85%, at least 90%, at least 95%, at least 98%, at least 99% or 100% sequence identity to the polypeptide shown in SEQ ID NO: 97,
vi) a polypeptide having at least 80%, at least 85%, at least 90%, at least 95%, at least 98%, at least 99% or 100% sequence identity to the polypeptide shown in SEQ ID NO: 98,
vii) a polypeptide having at least 80%, at least 85%, at least 90%, at least 95%, at least 98%, at least 99% or 100% sequence identity to the polypeptide shown in SEQ ID NO: 99,
viii) a polypeptide having at least 80%, at least 85%, at least 90%, at least 95%, at least 98%, at least 99% or 100% sequence identity to the polypeptide shown in SEQ ID NO: 100,
ix) a polypeptide having at least 80%, at least 85%, at least 90%, at least 95%, at least 98%, at least 99% or 100% sequence identity to the polypeptide shown in SEQ ID NO: 101,
x) a polypeptide having at least 80%, at least 85%, at least 90%, at least 95%, at least 98%, at least 99% or 100% sequence identity to the polypeptide shown in SEQ ID NO: 102,
xi) a polypeptide having at least 80%, at least 85%, at least 90%, at least 95%, at least 98%, at least 99% or 100% sequence identity to the polypeptide shown in SEQ ID NO: 103,
xii) a polypeptide having at least 80%, at least 85%, at least 90%, at least 95%, at least 98%, at least 99% or 100% sequence identity to the polypeptide shown in SEQ ID NO: 104,

and wherein the DNase comprises a polypeptide having at least 80%, at least 85%, at least 90%, at least 95%, at least 98%, at least 99% or 100% sequence identity to the amino acid sequence shown in SEQ ID NO 13.

The invention further relates to a method of deep cleaning of an item, comprising the steps of:

a) contacting the item with a cleaning composition comprises a DNase, an RNase and a cleaning component; and
b) and optionally rinsing the item, wherein the item is preferably a textile.

The invention further relates to a method of deep cleaning of an item, comprising the steps of:

a) contacting the item with a cleaning composition comprises a DNase, wherein the DNase comprises a polypeptide having at least 80%, at least 85%, at least 90%, at least 95%, at least 98%, at least 99% or 100% sequence identity to the amino acid sequence shown in SEQ ID NO 13, and an RNase, wherein the RNase selected from the group consisting of polypeptides comprising;

i) a polypeptide having at least 80%, at least 85%, at least 90%, at least 95%, at least 98%, at least 99% or 100% sequence identity to the polypeptide shown in SEQ ID NO: 87,
ii) a polypeptide having at least 80%, at least 85%, at least 90%, at least 95%, at least 98%, at least 99% or 100% sequence identity to the polypeptide shown in SEQ ID NO: 90,
iii) a polypeptide having at least 80%, at least 85%, at least 90%, at least 95%, at least 98%, at least 99% or 100% sequence identity to the polypeptide shown in SEQ ID NO: 95,
iv) a polypeptide having at least 80%, at least 85%, at least 90%, at least 95%, at least 98%, at least 99% or 100% sequence identity to the polypeptide shown in SEQ ID NO: 96,
v) a polypeptide having at least 80%, at least 85%, at least 90%, at least 95%, at least 98%, at least 99% or 100% sequence identity to the polypeptide shown in SEQ ID NO: 97,
vi) a polypeptide having at least 80%, at least 85%, at least 90%, at least 95%, at least 98%, at least 99% or 100% sequence identity to the polypeptide shown in SEQ ID NO: 98,
vii) a polypeptide having at least 80%, at least 85%, at least 90%, at least 95%, at least 98%, at least 99% or 100% sequence identity to the polypeptide shown in SEQ ID NO: 99,
viii) a polypeptide having at least 80%, at least 85%, at least 90%, at least 95%, at least 98%, at least 99% or 100% sequence identity to the polypeptide shown in SEQ ID NO: 100,
ix) a polypeptide having at least 80%, at least 85%, at least 90%, at least 95%, at least 98%, at least 99% or 100% sequence identity to the polypeptide shown in SEQ ID NO: 101,
x) a polypeptide having at least 80%, at least 85%, at least 90%, at least 95%, at least 98%, at least 99% or 100% sequence identity to the polypeptide shown in SEQ ID NO: 102,
xi) a polypeptide having at least 80%, at least 85%, at least 90%, at least 95%, at least 98%, at least 99% or 100% sequence identity to the polypeptide shown in SEQ ID NO: 103, and
xii) a polypeptide having at least 80%, at least 85%, at least 90%, at least 95%, at least 98%, at least 99% or 100% sequence identity to the polypeptide shown in SEQ ID NO: 104, and a cleaning component; and

b) optionally rinsing the item, wherein the item is preferably a textile.

The invention further relates to a kit intended for deep cleaning, wherein the kit comprises a solution of an enzyme mixture comprising a DNase and an RNase.

The DNase is a polypeptide having at least 80%, at least 85%, at least 90%, at least 95%, at least 98%, at least 99% or 100% sequence identity to the amino acid sequence shown in SEQ ID NO 13, and the RNase is selected from the group consisting of polypeptides comprising;

i) a polypeptide having at least 80%, at least 85%, at least 90%, at least 95%, at least 98%, at least 99% or 100% sequence identity to the polypeptide shown in SEQ ID NO: 87,
ii) a polypeptide having at least 80%, at least 85%, at least 90%, at least 95%, at least 98%, at least 99% or 100% sequence identity to the polypeptide shown in SEQ ID NO: 90,
iii) a polypeptide having at least 80%, at least 85%, at least 90%, at least 95%, at least 98%, at least 99% or 100% sequence identity to the polypeptide shown in SEQ ID NO: 95,
iv) a polypeptide having at least 80%, at least 85%, at least 90%, at least 95%, at least 98%, at least 99% or 100% sequence identity to the polypeptide shown in SEQ ID NO: 96,
v) a polypeptide having at least 80%, at least 85%, at least 90%, at least 95%, at least 98%, at least 99% or 100% sequence identity to the polypeptide shown in SEQ ID NO: 97,
vi) a polypeptide having at least 80%, at least 85%, at least 90%, at least 95%, at least 98%, at least 99% or 100% sequence identity to the polypeptide shown in SEQ ID NO: 98,
vii) a polypeptide having at least 80%, at least 85%, at least 90%, at least 95%, at least 98%, at least 99% or 100% sequence identity to the polypeptide shown in SEQ ID NO: 99,
viii) a polypeptide having at least 80%, at least 85%, at least 90%, at least 95%, at least 98%, at least 99% or 100% sequence identity to the polypeptide shown in SEQ ID NO: 100,
ix) a polypeptide having at least 80%, at least 85%, at least 90%, at least 95%, at least 98%, at least 99% or 100% sequence identity to the polypeptide shown in SEQ ID NO: 101,
x) a polypeptide having at least 80%, at least 85%, at least 90%, at least 95%, at least 98%, at least 99% or 100% sequence identity to the polypeptide shown in SEQ ID NO: 102,
xi) a polypeptide having at least 80%, at least 85%, at least 90%, at least 95%, at least 98%, at least 99% or 100% sequence identity to the polypeptide shown in SEQ ID NO: 103, and
xii) a polypeptide having at least 80%, at least 85%, at least 90%, at least 95%, at least 98%, at least 99% or 100% sequence identity to the polypeptide shown in SEQ ID NO: 104.

## Definitions

### Nomenclature

For purposes of the present invention, the nomenclature [E/Q] means that the amino acid at this position may be a glutamic acid (Glu, E) or a glutamine (Gln, Q). Likewise, the nomenclature [V/G/A/I] means that the amino acid at this position may be a valine (Val, V), glycine (Gly, G), alanine (Ala, A) or isoleucine (Ile, I), and so forth for other combinations as described herein. Unless otherwise limited further, the amino acid X is defined such that it may be any of the 20 natural amino acids.

The term "biofilm" is produced by any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, RNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium. Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of the community. On laundry biofilm producing bacteria can be found among the following species: *Acinetobacter sp., Aeromicrobium sp., Brevundimonas sp., Microbacterium sp., Micrococcus luteus, Pseudomonas sp., Staphylococcus epidermidis, and Stenotrophomonas sp.* On hard surfaces biofilm producing bacteria can be found among the following species: *Acinetobacter sp., Aeromicrobium sp., Brevundimonas sp., Microbacterium sp., Micrococcus luteus, Pseudomonas sp., Staphylococcus epidermidis, Staphylococcus aureus and Stenotrophomonas sp.* In one aspect, the biofilm producing strain is *Brevundimonas sp.* In one aspect, the biofilm producing strain is *Pseudomonas alcaliphila* or *Pseudomonas fluorescens.* In one aspect, the biofilm producing strain is Staphylococcus aureus.

By the term "deep cleaning" is meant disruption or removal of components of organic matter, e.g. biofilm, such

as DNA, RNA, proteins, polysaccharides or other components present in the organic matter.

Cleaning component: The cleaning component e.g. the detergent adjunct ingredient is different to the DNase and RNase enzymes. The precise nature of these additional cleaning components e.g. adjunct components, and levels of incorporation thereof, will depend on the physical form of the composition and the nature of the operation for which it is to be used. Suitable cleaning components e.g. adjunct materials include, but are not limited to the components described below such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

Cleaning composition: The term "cleaning composition" refers to compositions that find use in the removal of undesired compounds from items to be cleaned, such as textiles. The cleaning composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). In addition to containing the enzymes, the cleaning composition may contain one or more additional enzymes (such as amylases, lipases, cutinases, cellulases, endoglucanases, xyloglucanases, pectinases, pectin lyases, xanthanases, peroxidases, haloperoxygenases, catalases and mannanases, or any mixture thereof), and/or cleaning components e.g. detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and/or cleaning, prevention or reduction of redeposition of soils released in the washing process (an effect that also is termed anti-redeposition), restoring fully or partly the whiteness of textiles which originally were white but after repeated use and wash have obtained a greyish or yellowish appearance (an effect that also is termed whitening). Textile care benefits, which are not directly related to catalytic stain removal or prevention of redeposition of soils, are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric-softness, colour clarification of the fabric and removal of particulate soils which are trapped in the fibers of the fabric or garment. Enzymatic bleaching is a further enzyme detergency benefit where the catalytic activity generally is used to catalyze the formation of bleaching components such as hydrogen peroxide or other peroxides. Textile care benefits, which are not directly related to catalytic stain removal or prevention of redeposition of soils, are also important for enzyme detergency benefits. Examples of such textile care benefits are prevention or reduction of dye transfer from one textile to another textile or another part of the same textile (an effect that is also termed dye transfer inhibition or anti-backstaining), removal of protruding or broken fibers from a textile surface to decrease pilling tendencies or remove already existing pills or fuzz (an effect that also is termed anti-pilling), improvement of the textile-softness, colour clarification of the textile and removal of particulate soils which are trapped in the fibers of the textile. Enzymatic bleaching is a further enzyme detergency benefit where the catalytic activity generally is used to catalyze the formation of bleaching component such as hydrogen peroxide or other peroxides or other bleaching species."

The term "Hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, cutlery such as spoons, knives, forks, serving utensils, ceramics, plastics, metals, china, glass and acrylics.

The term "wash performance" is used as an enzyme's ability to remove stains present on the object to be cleaned during e.g. wash or hard surface cleaning.

The term "Whiteness" is defined herein as a greying, yellowing of a textile. Loss of whiteness may be due to removal of optical brighteners/hueing agents. Greying and yellowing can be due to soil redeposition, body soils, colouring from e.g. iron and copper ions or dye transfer. Whiteness might include one or several issues from the list below: colourant or dye effects; incomplete stain removal (e.g. body soils, sebum etc.); redeposition (greying, yellowing or other discolourations of the object) (removed soils reassociate with other parts of textile, soiled or unsoiled); chemical changes in textile during application; and clarification or brightening of colours.

The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell, which may be produced by microorganisms. Another example is unpleasant smells can be sweat or body odor adhered to an item which has been in contact with human or animal. Another example of malodor can be the odor from spices, which sticks to items for example curry or other exotic spices which smells strongly.

The term "mature polypeptide" means a polypeptide in its final form following translation and any post-translational modifications, such as N-terminal processing, C-terminal truncation, glycosylation, phosphorylation, etc.

Sequence identity: The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used, it is intended to include the broader term textiles as well.

The term "variant" means a polypeptide having the activity of the parent or precursor polypeptide and comprising an alteration, i.e., a substitution, insertion, and/or deletion, at one or more (e.g., several) positions compared to the precursor or parent polypeptide. A substitution means replacement of the amino acid occupying a position with a different amino acid; a deletion means removal of the amino acid occupying a position; and an insertion means adding an amino acid adjacent to and immediately following the amino acid occupying a position.

## Examples

Assays

Assay I: testing of DNase activity

DNase activity was determined on DNase Test Agar with Methyl Green (BD, Franklin Lakes, NJ, USA), which was prepared according to the manual from supplier. Briefly, 21 g of agar was dissolved in 500 ml water and then autoclaved for 15 min at 121°C. Autoclaved agar was temperated to 48°C in water bath, and 20 ml of agar was poured into petridishes with and allowed to solidify by incubation o/n at room temperature. On solidified agar plates, 5 μl of enzyme solutions are added and DNase activity is observed as colorless zones around the spotted enzyme solutions.

### Assay Ia

DNase activity may be determined by fluorescence using a fluorescence-quenched DNA oligonucleotide probe. This probe emits a signal after nuclease degradation according to the manual from the supplier (DNase alert kit, Integrated

DNA Technology, Coralville, Iowa, USA). Briefly, 5μl of the substrate is added to 95 μl of DNase. If the signal is too high, further dilutions of DNase are performed in a suitable buffer. Kinetic curves are measured for 20 min at 22°C using a Clariostar microplate reader (536 nm excitation, 556 nm emission).

**Assay II: testing RNase activity**

RNase activity may be determined by fluorescence using fluorescence-quenched oligonucleotides probe. This probe emits signal after nuclease degradation according to the manual from the supplier (RNase alert kit, Integrated DNA Technology, Coralville, Iowa, USA). Briefly, RNase is diluted in water hardness 15°dH to obtain a concentration of 2 ppm, 5μl of the substrate was added to 95 μl of the RNase sample. Kinetic curve was measured for 10 min at 22°C using a Clariostar microplate reader (excitation 490nm, emission at 520nm).

**Example 1:**

Isolating laundry specific bacterial strains

One strain of *Brevundimonas* sp. isolated from laundry was used in the present example. The *Brevundimonas* sp. was isolated during a study, where the bacterial diversity in laundry after washing at 15, 40 and 60°C, respectively, was investigated. The study was conducted on laundry collected from Danish households. For each wash, 20 g of laundry items (tea towel, towel, dish cloth, bib, T-shirt armpit, T-shirt collar, socks) in the range 4:3:2:2:1:1:1 was used. Washing was performed in a Laundr-O-Meter (LOM) at 15, 40 or 60°C. For washing at 15 and 40°C, Ariel Sensitive White & Color was used, whereas WFK IEC-A* model detergent was used for washing at 60°C. Ariel Sensitive White & Color was prepared by weighing out 5.1 g and adding tap water up to 1000 ml followed by stirring for 5 minutes. WFK IEC-A* model detergent (which is available from WFK Testgewebe GmbH) was prepared by weighing out 5 g and adding tap water up to 1300 ml followed by stirring for 15 min. Washing was performed for 1 hour at 15, 40 and 60°C, respectively, followed by 2 times rinsing with tap water for 20 min at 15°C.

Laundry was sampled immediately after washing at 15, 40 and 60°C, respectively. Twenty grams of laundry was added 0.9% (w/v) NaCl (1.06404; Merck, Darmstadt, Germany) with 0.5% (w/w) tween 80 to yield a 1:10 dilution in stomacher bag. The mixture was homogenized using a Stomacher for 2 minutes at medium speed. After homogenization, ten-fold dilutions were prepared in 0.9% (w/v) NaCl. Bacteria were enumerated on Tryptone Soya Agar (TSA) (CM0129, Oxoid, Basingstoke, Hampshire, UK) incubated aerobically at 30°C for 5-7 days. To suppress growth of yeast and moulds, 0.2% sorbic acid (359769, Sigma) and 0.1% cycloheximide (18079; Sigma) were added. Bacterial colonies were selected from countable plates and purified by restreaking twice on TSA. For long time storage, purified isolates were stored at -80°C in TSB containing 20% (w/v) glycerol (49779; Sigma).

Preparation of swatches with biofilm

Swatches with biofilm of *Brevundimonas* sp. were included in the present study. Bacteria was pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB and incubated for 1 day at 30°C with shaking (240 rpm). After propagation, cells were pelleted by centrifugation (Sigma Laboratory Centrifuge 6K15) (3000 g at 21°C in 7 min) and resuspended in 10 mL of TSB diluted twice with water. Optical density (OD) at 600 nm was measured using a spectophometer (POLARstar Omega (BMG Labtech, Ortenberg, Germany). Fresh TSB diluted twice with water was inoculated to an $OD_{600nm}$ of 0.03, and 50 mL was added into a petridish (diameter 125 mm), in which a swatch (80 mm × 120 mm) of either sterile cotton (WFK10A) or sterile polyester (WFK30A). After incubation (48 h at 15°C with shaking (100 rpm), swatches were rinsed twice with 0.9% (w/v) NaCl and dried in LAF bench for 60 min. Swatches were stored at 4°C prior to wash.

Wash experiment

Wash experiments were performed using the Automatic Mechanical Stress Assay (AMSA). With AMSA, the wash performance of many small volume enzyme-detergent solutions can be examined at the same time. The AMSA plate has many slots for test solutions, and a lid that firmly squeezes the textile to be washed against the slot openings. During the wash, the plate, test solutions, textile and lid are vigorously shaken to bring the test solution in contact with the textile and apply mechanical stress in a regular, periodic, oscillating manner.

The wash experiment was conducted under the experimental conditions specified below:

| | |
|---|---|
| Detergent dosage | 3.3 g/L (liquid detergent) |
| Test solution volume | 160 micro L |
| pH | pH 8 |
| Wash time | 20 minutes |
| Temperature | 30°C |
| Water hardness | 15°dH |
| Soil | Wfk09V 0.7g/L |

Model detergents and test materials were as follows:

| | |
|---|---|
| Laundry liquid model detergent | Model detergent A |
| Test material | Brevundimonas sp. 2-day biofilm grown on WFK10 (cotton) or WFK30A (polyester) |

Model detergent A (containing 12% LAS, 11% AEO Biosoft N25-7 (NI), 7% AEOS (SLES), 6% MPG, 3% ethanol, 3% TEA, 2.75% cocoa soap, 2.75% soya soap, 2% glycerol, 2% sodium hydroxide, 2% sodium citrate, 1% sodium formiate, 0.2% DTMPA and 0.2% PCA (all percentages are w/w)) (3.3 g/L) dissolved in water hardness 15°dH (Ca:Mg:NaHCOs = 4:1:1.5) was used. Soil was subsequently added to reach a concentration of 0.7 g soil/L (WFK09V pigment soil) to reveal biofilm. After washing, textiles were flushed in tap water and dried over night before scanning. Experiments were done twice.

Wash performance was measured as the brightness of the WFK09V pigment soiled, washed textile. Brightness can also be expressed as the intensity of the light reflected from the sample when illuminated with white light. When the sample is soiled, the intensity of the reflected light is lower, than that of a clean sample. Therefore, the intensity of the reflected light can be used to measure wash performance. Intensity measurements were made with a professional flatbed scanner (Kodak iQsmart, Kodak, Midtager 29, DK-2605 Brøndby, Denmark), which was used to capture an image of the washed and dried textile. To extract a value for the light intensity from the scanned images, 24-bit pixel values from the image were converted into values for red, green and blue (RGB). The intensity value (Int) was calculated by adding the RGB values together as vectors and then taking the length of the resulting vector:

$$Int = \sqrt{r^2 + g^2 + b^2}$$

To assess wash synergy between DNase (SEQ ID NO 13) and RNase (SEQ ID NO 87), biofilm-harbouring textile was AMSA washed a) in the absence of enzyme (blank), b) in the presence of DNase alone and c) with a mixture of DNase and RNase. The resulting textile intensities and corresponding wash performances (WPs) are listed in Table 1, 2 and 3. Wash performances attributable to DNase ($WP_{DNase}$) and the mixture of the two ($WP_{DNase+RNase}$) were quantified as the difference in intensity between textile washed with and without enzyme:

$$WP_{DNase} = I_{DNase} - I_{Blank},\ WP_{DNase+RNase} = I_{DNase+RNase} - I_{Blank}.$$

Table 1. Synergistic wash effect of DNase and RNase (cotton swatches) (experiment 1)

| | | I | WP |
|---|---|---|---|
| Blank | No enzyme | 303.506 | - |
| DNase | 0.00002 ppm DNase | 314.331 | 10.83 |
| DNase+RNase | 0.00002 ppm DNase + 0.2 ppm RNase | 318.246 | 14.740 |
| | 0.00002 ppm DNase + 2 ppm RNase | 322.057 | 18.551 |

Table 2. Synergistic wash effect of DNase and RNase (cotton swatches) (experiment 2)

| | | I | WP |
|---|---|---|---|
| Blank | No enzyme | 303.506 | - |
| DNase | 0.00002 ppm DNase | 314.331 | 10.83 |
| DNase+RNase | 0.00002 ppm DNase + 0.2 ppm RNase | 316.686 | 13.180 |
| | 0.00002 ppm DNase + 2 ppm RNase | 322.917 | 19.411 |

Table 3. Synergistic wash effect of DNase and RNase (polyester swatches) (experiment 1 + 2)

| | | I | WP |
|---|---|---|---|
| Blank | No enzymes | 301.805 | - |
| DNase | 0.00002 ppm DNase | 323.358 | 21.55 |
| DNase+RNase | 0.00002 ppm DNase + 0.02 ppm RNase | 332.049 | 30.244 |
| | | I | WP |
| Blank | No enzymes | 301.805 | - |
| DNase | 0.00002 ppm DNase | 323.358 | 21.55 |
| DNase+RNase | 0.00002 ppm DNase + 0.02 ppm RNase | 330.988 | 29.182 |

The results shown above show that adding an RNase to a cleaning composition comprising a DNase can boost the deep cleaning performance of the DNase in laundry.

**Example 2**

Preparation of biofilm swatches:

*Brevundimonas* sp. was pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB and incubated for 1 day at 30°C with shaking (240 rpm). After propagation, *Brevundimonas* sp. was pelleted by centrifugation (Sigma Laboratory Centrifuge 6K15) (3000 g at 21°C in 7 min) and resuspended in 10 mL of TSB diluted twice with water. Optical density (OD) at 600 nm was measured using a spectophometer (CLARIOstar Omega (BMG Labtech, Ortenberg, Germany). Fresh TSB diluted twice with water was inoculated to an $OD_{600nm}$ of 0.03, and 20 mL was added into petridish, in which a swatch (8 × 12 cm) of sterile Cotton WFK10A was placed. After incubation (72 h at 15°C with shaking (100 rpm), swatches were rinsed twice with 0.9% (w/v) NaCl and dried and stored at 4°C until use.

**Preparation of extracts:**

Biofilm extracts

Brevundimonas biofilm swatches were cut into 0.5 cm × 0.5 cm pieces and mixed thoroughly. 1 g was weighed out in a 50 ml tubes and 30 ml of extraction buffer (0.9 (w/w) % NaCl; 10 mM EDTA) was added to each tube and placed in a Stuart rotator for 60 min at 40 rpm. Afterwards, tubes were centrifuged 4000 rpm for 10 min at 20°C to remove textile. Supernatants were collected from each tube and pooled. Supernatant was further clarified by filtration through a 0.2 μm filter. Before use, filtrated extract was diluted 75 times.

Extracts of real items - heavy soiled pillowcases from Warwick Equest

2 g of swatches (0.5 cm × 0.5 cm) from the middle of three different pillowcases were placed into 50 ml tubes and 30 ml of extraction buffer (0.9 (w/w) % NaCl; 10 mM EDTA) were added to each tube and placed in a Stuart rotator for 60 min at 40 rpm. Afterwards, tubes were centrifuged 4000 rpm for 10 min at 20°C to remove textile. Supernatants were collected from each tube and pooled. Supernatant was further clarified by filtration through a 0.2 μm filter.

Measurement of boosting effects:

Residual DNA in extracts was measured with Quant-iT™ PicoGreen™ dsDNA Assay Kit (P7589; ThermoFisher Scientific) using a fluorometer (CLARIOstar Omega Clariostar Omega (BMG Labtech, Ortenberg, Germany).

Following solutions were prepared:

| Name | Procedure | Storage |
|---|---|---|
| 1xTE buffer | 20-fold dilution of the TE buffer provided in kit (e.g. 5mL TE buffer to 95mL sterile DNAse-free water) | Up to 6 months at room temperature |
| Assay reagent | 200-fold dilution of concentrated reagent from kit. (e.g. 100μL reagent to 20mL 1xTE buffer) | Pack in tinfoil, use within hours. |
| 2×15°dH water | Prepare 1L with 4:1 Ca/Mg ratio:<br>1. Add 0.674g NaHCO3 and a magnet to a jug<br>2. Add 1000mL Milli-Q water<br>3. Add 6.0mL 4000°dH CaCl2 stock<br>4. Add 3.0mL MgCl2 stock<br>5. Stir for a minimum of 10min | Prepare and use on the day of the experiment. |

In a well of a black microtiter plate, 50μl of extract from biofilm and pillowcases, respectively, was adjusted resulting in water hardness 15°dH, and 100μl picogreen (dilution 200x) was added. Enzyme was added resulting in a conc. of 0.2 ppm and incubated for 1h at room temperature. Fluorescence was measured at excitation 483-15nm/emission 530-30 nm.

Table 4. Boosting on biofilm extracts of RNases in combination with DNase (SEQ ID NO 13).

| RNase | Rest DNA (RNase) | Rest DNA (DNase) | Rest DNA (RNase + DNase) | Additional DNA removal for combination |
|---|---|---|---|---|
| SEQ ID NO 96 | 84 | 20 | 16 | 4 |
| SEQ ID NO 97 | 87 | 20 | 11 | 9 |
| SEQ ID NO 98 | 83 | 20 | 13 | 8 |
| SEQ ID NO 99 | 95 | 20 | 10 | 11 |
| SEQ ID NO 103 | 65 | 20 | 12 | 8 |
| SEQ ID NO 90 | 76 | 20 | 10 | 10 |
| SEQ ID NO 100 | 87 | 20 | 13 | 8 |
| SEQ ID NO 101 | 90 | 20 | 15 | 6 |
| SEQ ID NO 102 | 85 | 20 | 11 | 9 |
| SEQ ID NO 104 | 84 | 20 | 15 | 6 |

Table 5. Boosting effects on pillowcase extracts of RNases in combination with DNase (SEQ ID NO 13).

| RNase | Rest DNA (RNase) | Rest DNA (DNase) | Rest DNA (RNase + DNase) | Additional DNA removal for combination |
|---|---|---|---|---|
| SEQ ID NO 95 | 91 | 52 | 40 | 12 |
| SEQ ID NO 96 | 83 | 52 | 39 | 13 |
| SEQ ID NO 97 | 76 | 52 | 38 | 14 |
| SEQ ID NO 98 | 72 | 52 | 36 | 16 |
| SEQ ID NO 99 | 76 | 52 | 38 | 14 |
| SEQ ID NO 103 | 78 | 52 | 37 | 14 |
| SEQ ID NO 90 | 83 | 52 | 39 | 13 |
| SEQ ID NO 100 | 85 | 52 | 43 | 9 |
| SEQ ID NO 102 | 94 | 52 | 49 | 3 |
| SEQ ID NO 104 | 88 | 52 | 41 | 11 |

All the tested RNases, when added to a cleaning composition comprising a DNase boost the deep cleaning performance of the DNase in laundry.

## Claims

1. A cleaning composition comprising a DNase, an RNase and a cleaning component, wherein the DNase comprises one or both of the motifs [D/M/L][S/T]GYSR[D/N] (SEQ ID NO: 73) or ASXNRSKG (SEQ ID NO: 74) and comprises an amino acid sequence having at least 80% sequence identity to SEQ ID NO: 13, and wherein the RNase comprises an amino acid sequence having at least 80% sequence identity to SEQ ID NO: 87, SEQ ID NO: 90, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103 or SEQ ID NO: 104.

2. The cleaning composition according to claim 1, wherein the DNase comprises a polypeptide having at least 85%, at least 90%, at least 95%, at least 98%, at least 99% or 100% sequence identity to the amino acid sequence shown in SEQ ID NO 13.

3. The cleaning composition according to claim 1 or 2, wherein the RNase is selected from the group consisting of RNases comprising an amino acid sequence with:

   i) at least 80%, at least 85%, at least 90%, at least 95%, at least 98%, at least 99% or 100% sequence identity to the polypeptide shown in SEQ ID NO: 87,
   ii) at least 80%, at least 85%, at least 90%, at least 95%, at least 98%, at least 99% or 100% sequence identity to the polypeptide shown in SEQ ID NO: 90,
   iii) at least 80%, at least 85%, at least 90%, at least 95%, at least 98%, at least 99% or 100% sequence identity to the polypeptide shown in SEQ ID NO: 95,
   iv) at least 80%, at least 85%, at least 90%, at least 95%, at least 98%, at least 99% or 100% sequence identity

to the polypeptide shown in SEQ ID NO: 96,
v) at least 80%, at least 85%, at least 90%, at least 95%, at least 98%, at least 99% or 100% sequence identity to the polypeptide shown in SEQ ID NO: 97,
vi) at least 80%, at least 85%, at least 90%, at least 95%, at least 98%, at least 99% or 100% sequence identity to the polypeptide shown in SEQ ID NO: 98,
vii) at least 80%, at least 85%, at least 90%, at least 95%, at least 98%, at least 99% or 100% sequence identity to the polypeptide shown in SEQ ID NO: 99,
viii) at least 80%, at least 85%, at least 90%, at least 95%, at least 98%, at least 99% or 100% sequence identity to the polypeptide shown in SEQ ID NO: 100,
ix) at least 80%, at least 85%, at least 90%, at least 95%, at least 98%, at least 99% or 100% sequence identity to the polypeptide shown in SEQ ID NO: 101,
x) at least 80%, at least 85%, at least 90%, at least 95%, at least 98%, at least 99% or 100% sequence identity to the polypeptide shown in SEQ ID NO: 102,
xi) at least 80%, at least 85%, at least 90%, at least 95%, at least 98%, at least 99% or 100% sequence identity to the polypeptide shown in SEQ ID NO: 103, and
xii) at least 80%, at least 85%, at least 90%, at least 95%, at least 98%, at least 99% or 100% sequence identity to the polypeptide shown in SEQ ID NO: 104.

**4.** The cleaning composition according to any of the preceding claims, wherein the amount of DNase in the composition is from 0.01 to 1000 ppm and the amount of RNase is from 0.01 to 1000 ppm.

**5.** The cleaning composition according to any of the preceding claims, wherein the cleaning component is selected from surfactants, preferably anionic and/or nonionic, builders and bleach components.

**6.** Use of a cleaning composition according to any of claims 1 to 5 for deep cleaning of an item, wherein the item is a textile or a surface.

**7.** A method of formulating a cleaning composition according to any of claims 1 to 5 comprising adding a DNase, an RNase and at least one cleaning component, wherein the DNase comprises one or both of the motifs [D/M/L][S/T]GYSR[D/N] (SEQ ID NO: 73) or ASXNRSKG (SEQ ID NO: 74) and comprises an amino acid sequence having at least 80% sequence identity to SEQ ID NO 13, and wherein the RNase comprises an amino acid sequence having at least 80% sequence identity to SEQ ID NO: 87, SEQ ID NO: 90, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103 or SEQ ID NO: 104.

**8.** A kit intended for deep cleaning, wherein the kit comprises a solution of an enzyme mixture comprising a DNase, an RNase and optionally a protease, wherein the DNase comprises one or both of the motifs [D/M/L][S/T]GYSR[D/N] (SEQ ID NO: 73) or ASXNRSKG (SEQ ID NO: 74) ) and comprises an amino acid sequence having at least 80% sequence identity to SEQ ID NO 13, and wherein the RNase comprises an amino acid sequence having at least 80% sequence identity to SEQ ID NO: 87, SEQ ID NO: 90, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103 or SEQ ID NO: 104.

**9.** A method of deep cleaning of an item, comprising the steps of:

a) contacting the item with a cleaning composition according to any of claims 1 to 5; and optionally,
b) rinsing the item,

wherein the item is preferably a textile.

**Patentansprüche**

**1.** Reinigungszusammensetzung, die eine DNase, eine RNase und einen Reinigungsbestandteil umfasst, wobei die DNase eines oder beide der Motive [D/M/L][S/T]GYSR[D/N] (SEQ ID NO: 73) oder ASXNRSKG (SEQ ID NO: 74) umfasst und eine Aminosäuresequenz mit mindestens 80% Sequenzidentität zu SEQ ID NO: 13 umfasst, und wobei die RNase eine Aminosäuresequenz mit mindestens 80% Sequenzidentität zu SEQ ID NO: 87, SEQ ID NO: 90, SEQ ID NO: 95, SEQ ID NO: 96, SE Q ID NO: 97, SEQ ID NO: 98, SEQ ID NO: 99, SEQ ID NO: 100, SEQ ID NO: 101, SEQ ID NO: 102, SEQ ID NO: 103 oder SEQ ID NO: 104 umfasst.

**2.** Reinigungszusammensetzung nach Anspruch 1, wobei die DNase ein Polypeptid mit mindestens 85%, mindestens 90%, mindestens 95%, mindestens 98%, mindestens 99% oder 100% Sequenzidentität zu der in SEQ ID NO: 13 gezeigten Aminosäuresequenz umfasst.

**3.** Reinigungszusammensetzung nach Anspruch 1 oder 2, wobei die RNase aus der Gruppe ausgewählt ist, die aus RNasen besteht, die eine Aminosäuresequenz umfassen mit:

i) mindestens 80%, mindestens 85%, mindestens 90%, mindestens 95%, mindestens 98%, mindestens 99% oder 100% Sequenzidentität zu dem in SEQ ID NO: 87 gezeigten Polypeptid,
ii) mindestens 80%, mindestens 85%, mindestens 90%, mindestens 95%, mindestens 98%, mindestens 99% oder 100% Sequenzidentität zu dem in SEQ ID NO: 90 gezeigten Polypeptid,
iii) mindestens 80%, mindestens 85%, mindestens 90%, mindestens 95%, mindestens 98%, mindestens 99% oder 100% Sequenzidentität zu dem in SEQ ID NO: 95 gezeigten Polypeptid,
iv) mindestens 80%, mindestens 85%, mindestens 90%, mindestens 95%, mindestens 98%, mindestens 99% oder 100% Sequenzidentität zu dem in SEQ ID NO: 96 gezeigten Polypeptid,
v) mindestens 80%, mindestens 85%, mindestens 90%, mindestens 95%, mindestens 98%, mindestens 99% oder 100% Sequenzidentität zu dem in SEQ ID NO: 97 gezeigten Polypeptid,
vi) mindestens 80%, mindestens 85%, mindestens 90%, mindestens 95%, mindestens 98%, mindestens 99% oder 100% Sequenzidentität zu dem in SEQ ID NO: 98 gezeigten Polypeptid,
vii) mindestens 80%, mindestens 85%, mindestens 90%, mindestens 95%, mindestens 98%, mindestens 99% oder 100% Sequenzidentität zu dem in SEQ ID NO: 99 gezeigten Polypeptid,
viii) mindestens 80%, mindestens 85%, mindestens 90%, mindestens 95%, mindestens 98%, mindestens 99% oder 100% Sequenzidentität zu dem in SEQ ID NO: 100 gezeigten Polypeptid,
ix) mindestens 80%, mindestens 85%, mindestens 90%, mindestens 95%, mindestens 98%, mindestens 99% oder 100% Sequenzidentität zu dem in SEQ ID NO: 101 gezeigten Polypeptid,
x) mindestens 80%, mindestens 85%, mindestens 90%, mindestens 95%, mindestens 98%, mindestens 99% oder 100% Sequenzidentität zu dem in SEQ ID NO: 102 gezeigten Polypeptid,
xi) mindestens 80%, mindestens 85%, mindestens 90%, mindestens 95%, mindestens 98%, mindestens 99% oder 100% Sequenzidentität zu dem in SEQ ID NO: 103 gezeigten Polypeptid, und
xii) mindestens 80%, mindestens 85%, mindestens 90%, mindestens 95%, mindestens 98%, mindestens 99% oder 100% Sequenzidentität zu dem in SEQ ID NO: 104 gezeigten Polypeptid.

**4.** Reinigungszusammensetzung nach einem beliebigen der voranstehenden Ansprüche, wobei die Menge an DNase in der Zusammensetzung von 0,01 bis 1000 ppm beträgt, und die Menge an RNase von 0,01 bis 1000 ppm beträgt.

**5.** Reinigungszusammensetzung nach einem beliebigen der voranstehenden Ansprüche, wobei der Reinigungsbestandteil aus oberflächenaktiven Mitteln, bevorzugt anionisch und/oder nichtionisch, Gerüststoffen und Bleichbestandteilen ausgewählt ist.

**6.** Verwendung einer Reinigungszusammensetzung gemäß einem beliebigen der Ansprüche 1 bis 5 zum Tiefenreinigen eines Gegenstands, wobei der Gegenstand ein Textil oder eine Oberfläche ist.

**7.** Verfahren zum Formulieren einer Reinigungszusammensetzung gemäß einem beliebigen der Ansprüche 1 bis 5, umfassend Zugeben einer DNase, einer RNase und mindestens eines Reinigungsbestandteils, wobei die DNase eines oder beide der Motive [D/M/L][S/T]GYSR[D/N] (SEQ ID NO: 73) oder ASXNRSKG (SEQ ID NO: 74) umfasst, und eine Aminosäuresequenz mit mindestens 80% Sequenzidentität zu SEQ ID NO: 13 umfasst, und wobei die RNase eine Aminosäuresequenz mit mindestens 80% Sequenzidentität zu SEQ ID NO: 87, SEQ ID NO: 90, SEQ ID NO: 95, SEQ ID NO : 96, SEQ ID NO: 97, SEQ ID NO: 98, SEQ ID NO: 99, SEQ ID NO: 100, SEQ ID NO: 101, SEQ ID NO: 102, SEQ ID NO: 103 oder SEQ ID NO: 104 umfasst.

**8.** Kit, das für eine Tiefenreinigung vorgesehen ist, wobei das Kit eine Lösung einer Enzymmischung umfasst, die eine DNase, eine RNase und gegebenenfalls eine Protease umfasst, wobei die DNase eines oder beide der Motive [D/M/L][S/T]GYSR[D/N] (SEQ ID NO: 73) oder ASXNRSKG (SEQ ID NO: 74) umfasst, und eine Aminosäuresequenz mit mindestens 80% Sequenzidentität zu SEQ ID NO 13 umfasst, und wobei die RNase eine Aminosäuresequenz mit mindestens 80% Sequenzidentität zu SEQ ID NO: 87, SEQ ID NO: 90, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103 oder SEQ ID NO: 104 umfasst.

**9.** Verfahren zum Tiefenreinigen eines Gegenstands, umfassend die Schritte:

a) In-Kontakt-Bringen des Gegenstands mit einer Reinigungszusammensetzung gemäß einem beliebigen der Ansprüche 1 bis 5; und gegebenenfalls
b) Spülen des Gegenstands, wobei der Gegenstand bevorzugt ein Textil ist.

## Revendications

**1.** Composition nettoyante comprenant une DNase, une RNase et un composant nettoyant, dans laquelle la DNase comprend l'un ou les deux des motifs [D/M/L][S/T]GYSR[D/N] (SEQ ID NO : 73) et ASXNRSKG (SEQ ID NO : 74) et comprend une séquence d'acides aminés ayant une identité de séquence d'au moins 80 % avec la SEQ ID NO : 13, et dans laquelle la RNase comprend une séquence d'acides aminés ayant une identité de séquence d'au moins 80 % avec la SEQ ID NO : 87, la SEQ ID NO : 90, la SEQ ID NO : 95, la SEQ ID NO : 96, la SEQ ID NO : 97, la SEQ ID NO : 98, la SEQ ID NO : 99, la SEQ ID NO : 100, la SEQ ID NO : 101, la SEQ ID NO : 102, la SEQ ID NO : 103 ou la SEQ ID NO : 104.

**2.** Composition nettoyante selon la revendication 1, dans laquelle la DNase comprend un polypeptide ayant une identité de séquence d'au moins 85 %, d'au moins 90 %, d'au moins 95 %, d'au moins 98 %, d'au moins 99 % ou de 100 % avec la séquence d'acides aminés indiquée dans la SEQ ID NO : 13.

**3.** Composition nettoyante selon la revendication 1 ou 2, dans laquelle la RNase est choisie dans le groupe constitué par les RNases comprenant une séquence d'acides aminés ayant :

i) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 87,
ii) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 90,
iii) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 95,
iv) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 96,
v) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 97,
vi) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 98,
vii) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 99,
viii) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 100,
ix) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 101,
x) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 102,
xi) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 103, et
xii) une identité de séquence d'au moins 80 %, d'au moins 85 %, d'au moins 90 %, d'au moins 95 %, d'au moins 98 %, d'au moins 99 % ou de 100 % avec le polypeptide indiqué dans la SEQ ID NO : 104.

**4.** Composition nettoyante selon l'une quelconque des revendications précédentes, dans laquelle la quantité de DNase dans la composition est de 0,01 à 1000 ppm et la quantité de RNase est de 0,01 à 1000 ppm.

**5.** Composition nettoyante selon l'une quelconque des revendications précédentes, dans laquelle le composant nettoyant est choisi parmi les tensioactifs, de préférence anioniques et/ou non-ioniques, les adjuvants et les composants de blanchiment.

**6.** Utilisation d'une composition nettoyante selon l'une quelconque des revendications 1 à 5 pour le nettoyage en profondeur d'un article, dans laquelle l'article est un textile ou une surface.

**7.** Méthode de formulation d'une composition nettoyante selon l'une quelconque des revendications 1 à 5, comprenant l'addition d'une DNase, d'une RNase et d'au moins un composant nettoyant, dans laquelle la DNase comprend l'un ou les deux des motifs [D/M/L][S/T]GYSR[D/N] (SEQ ID NO : 73) et ASXNRSKG (SEQ ID NO : 74) et comprend une séquence d'acides aminés ayant une identité de séquence d'au moins 80 % avec la SEQ ID NO : 13, et dans laquelle la RNase comprend une séquence d'acides aminés ayant une identité de séquence d'au moins 80 % avec la SEQ ID NO : 87, la SEQ ID NO : 90, la SEQ ID NO : 95, la SEQ ID NO : 96, la SEQ ID NO : 97, la SEQ ID NO : 98, la SEQ ID NO : 99, la SEQ ID NO : 100, la SEQ ID NO : 101, la SEQ ID NO : 102, la SEQ ID NO : 103 ou la SEQ ID NO : 104.

**8.** Trousse destinée à un nettoyage en profondeur, lequel kit comprend une solution d'un mélange d'enzymes comprenant une DNase, une RNase et éventuellement une protéase, dans laquelle la DNase comprend l'un ou les deux des motifs [D/M/L][S/T]GYSR[D/N] (SEQ ID NO : 73) et ASXNRSKG (SEQ ID NO : 74) et comprend une séquence d'acides aminés ayant une identité de séquence d'au moins 80 % avec la SEQ ID NO : 13, et dans laquelle la RNase comprend une séquence d'acides aminés ayant une identité de séquence d'au moins 80 % avec la SEQ ID NO : 87, la SEQ ID NO : 90, la SEQ ID NO : 95, la SEQ ID NO : 96, la SEQ ID NO : 97, la SEQ ID NO : 98, la SEQ ID NO : 99, la SEQ ID NO : 100, la SEQ ID NO : 101, la SEQ ID NO : 102, la SEQ ID NO : 103 ou la SEQ ID NO : 104.

**9.** Méthode de nettoyage en profondeur d'un article, comprenant les étapes de :

a) mise en contact de l'article avec une composition nettoyante selon l'une quelconque des revendications 1 à 5 ; et éventuellement
b) rinçage de l'article,

dans laquelle l'article est de préférence un textile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016176241 A
- WO 2016176280 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 9426860 A
- WO 9426859 A
- WO 2006130575 A
- WO 200503274 A
- WO 200503275 A
- WO 200503276 A
- EP 1876226 A
- WO 2007087257 A
- WO 2007087243 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- US 5352604 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- WO 1999064619 A
- WO 9324618 A
- WO 9510602 A
- WO 9815257 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A

- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 13184577 A
- WO 10104675 A
- WO 2011098531 A
- WO 2013001078 A
- WO 2013001087 A
- EP 179486 A
- WO 9201046 A
- JP 2238885 A
- WO 9708325 A
- WO 9533836 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- US 4106991 A
- US 4661452 A
- GB 1483591 A
- EP 238216 A
- WO 2013188331 A
- WO 9307263 A
- WO 9723606 A
- WO 0001793 A
- WO 2006034710 A
- WO 9932595 A


**Non-patent literature cited in the description**


- Enzyme Nomenclature. NC-IUBMB, Academic Press, 1992
- *Eur. J. Biochem.,* 1994, vol. 1223 (1-5), 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **SIEZEN et al.** *Protein Engng.,* 1991, vol. 4, 719-737
- **SIEZEN et al.** *Protein Science,* 1997, vol. 6, 501-523
- Powdered Detergents. Surfactant science series. Marcel Dekker, Inc, vol. 71
- Particle size enlargement. **C. E. CAPES.** Handbook of Powder Technology. Elsevier, 1980, vol. 1
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277